# EUROPEAN PATENT SPECIFICATION

(11) **EP 0 528 146 B1**
(45) Date of publication and mention of the grant of the patent: **21.02.1996**
(21) Application number: 92110945.0
(22) Date of filing: 29.06.1992
(51) Int. Cl.: C07C 335/16, A61K 31/17, A61K 31/19, A61K 31/22, A61K 31/40, A61K 31/445, A61K 31/495, A61K 31/535, A61K 31/55

(54) **N-phenylthiourea derivatives and pharmaceutical use thereof**
Phenylthioharnstoffderivate und ihre pharmazeutische Verwendung
Dérivés de la phényl-thiourée et leur utilisation pharmaceutique

(30) Priority: 01.07.1991 US 724001
(43) Date of publication of application: 24.02.1993
(73) Proprietor: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Coppola, Gary Mark, Budd Lake, NJ 07828 (US); Damon, Robert Edson II, Wharton, NJ 07885 (US)

(56) References cited:
- EP-A- 0 297 610
- EP-A- 0 335 374
- EP-A- 0 392 802
- EP-A- 0 405 233
- GB-A- 2 113 684
- US-A- 3 950 537
- JOURNAL OF MEDICINAL CHEMISTRY vol. 32, no. 10, October 1989, WASHINGTON US pages 2318 - 2325 V.G. DEVRIES ET AL. 'Potential Antiatherosclerotic Agents. 6. Hypocholesterolemic Trisubstituted Urea Analogues'
- Liebigs Ann. Chem. 746, 54-64 (1971)

## Description

The invention relates to N-phenylthiourea derivatives and their pharmaceutical use.

It concerns the use of a compound of formula I 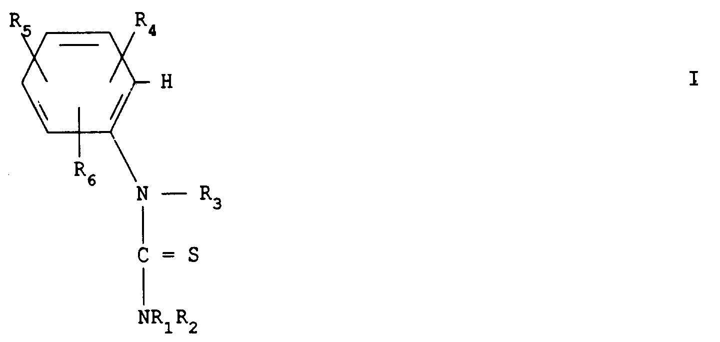
wherein
- R₁: is hydrogen; alkyl of 1 to 22 carbon atoms; cycloalkyl of 3 to 6 carbon atoms optionally mono- or independently disubstituted by alkyl of 1 or 2 carbon atoms; cycloalkylalkyl of 3 to 6 carbon atoms in the cycloalkyl and of 1 to 3 carbon atoms in the alkylene part or phenylalkyl of altogether 7 to 9 carbon atoms; hydroxyalkyl, alkoxyalkyl or mercaptoalkyl of 2 to 4 carbon atoms in the alkylene part, of 1 to 3 carbon atoms in the alkoxy part, and with the hydroxy, alkoxy or mercapto part attached in other than the 1-position; -CR₇R₈R₉ or -CH₂CR₇R₈R₉ wherein R₇ and R₈ either independently are hydrogen, methyl or ethyl or together are unbranched alkylene of 3 to 5 carbon atoms, and R₉ is carboxy, alkoxycarbonyl of altogether 2 to 4 carbon atoms or carbamoyl; alkenyl of 3 to 22 carbon atoms wherein the double bond is in other than the 1-position, or alkadienyl of 5 to 22 carbon atoms or alkatrienyl of 7 to 22 carbon atoms wherein each double bond is in other than the 1-position and no carbon atom is part of two double bonds, and
- R₂: is hydrogen;
- R₃: is hydrogen or alkyl of 1 to 3 carbon atoms;
- R₄: is hydrogen; alkyl of 1 to 4 carbon atoms; alkoxy of 1 to 3 carbon atoms; halogen of atomic number of from 9 to 35; trifluoromethyl; cyano; dialkylamino of independently 1 to 3 carbon atoms in the alkyl parts; nitro; phenyl; or benzyl;
- R₅: is hydrogen; alkyl of 1 to 4 carbon atoms; alkoxy of 1 to 3 carbon atoms; halogen of atomic number 9 or 17; or trifluoromethyl; and
- R₆: is hydrogen; alkyl of 1 to 3 carbon atoms; or alkoxy of 1 to 3 carbon atoms;
with the provisos that
i) when both R₄ and R₅ are trifluoromethyl, then they are not ortho to each other;
ii) when R₁ is hydroxyalkyl and each of R₄, R₅ and R₆ is selected from hydrogen and alkyl,
   then at least two of R₄, R₅ and R₆ are alkyl; and
iii) when R₁ is alkyl and R₄ is phenyl,
   then R₅ and R₆ are other than alkoxy;
   in free form or salt form as appropriate,
   for the manufacture of a medicament against atherosclerosis or of a medicament for raising blood serum high density lipoprotein level.

As appears from the formula a compound of formula I cannot be substituted in both positions on the phenyl ring ortho to the nitrogen moiety.

A compound of formula I may be in free form, e.g. as a base, or salt form, e.g. anionic or acid addition salt form where such forms exist, as appropriate. Preferred acid addition salt forms are pharmaceutically acceptable acid addition salt forms, preferred anionic salt forms are salts with pharmaceutically acceptable cations. Included are salts with organic acids, e.g. the methanesulfonate, ethanesulfonate, benzenesulfonate, p-toluenesulfonate, acetate and propionate salts, and salts with inorganic acids, e.g. the hydrochloride and sulfate salts.

R₁ preferably is alkyl, cycloalkyl, cycloalkylalkyl, alkoxyalkyl, alkenyl, or a group -CR₇R₈R₉ or -CH₂CR₇R₈R₉ as defined above. R₃ preferably is hydrogen. R₄ preferably is halogen, alkyl or benzyl, especially halogen or alkyl, particularly halogen. It preferably is in the 5-position. R₅ preferably is hydrogen, alkyl or alkoxy, especially alkyl. It preferably is in the 2-position. R₆ preferably is hydrogen or alkoxy, especially hydrogen. When it is other than hydrogen it preferably is in the 4-position. R₇ and R₈ preferably are methyl or ethyl, especially methyl. R₉ preferably is alkoxycarbonyl or carbamoyl.

Some of the compounds of formula I have one or more centers of asymmetry. Therefore, there are two or more enantiomers of each such compound. All of the possible isomers and racemates are within the scope of the invention. The compounds preferably are free of centers of asymmetry.

Alkyl of 1 to 22 carbon atoms preferably is of 1 to 8, especially of 3 to 5, particularly of 3 or 4 carbon atoms. When it is of more than 3 carbon atoms it preferably is branched, preferably at a position other than at the carbon atom attached to the nitrogen atom. It preferably is propyl or isopropyl, or of 4 to 22 carbon atoms and branched at a position other than at the carbon atom attached to the nitrogen atom. It especially is propyl, isopropyl or 2-methylpropyl. It particularly is 2-methylpropyl.

Cycloalkyl preferably is of 3 to 5, especially of 3 or 5 carbon atoms. It preferably is unsubstituted. Cycloalkylalkyl preferably is of 1 carbon atom in the alkylene part and preferably of 3 to 5, especially 3 carbon atoms in the cycloalkyl part. Phenylalkyl preferably is benzyl. The alkylene part of hydroxyalkyl, alkoxyalkyl or mercaptoalkyl preferably is of 2 or 3 carbon atoms; it preferably is unbranched.

Alkoxy as a substituent or part of a substituent preferably is methoxy. Unbranched alkylene conveniently is of 3 carbon atoms. Alkoxycarbonyl preferably is methoxycarbonyl. Alkenyl preferably is of 3 to 18 carbon atoms. Alkadienyl and alkatrienyl preferably are of 7 to 18 carbon atoms.

Alkyl of 1 to 4 or of 1 to 3 carbon atoms preferably is methyl or ethyl, especially methyl. The alkyl parts of dialkylamino or dialkylaminomethyl preferably are methyl. Alkylcarbonyl preferably is acetyl. Halogen preferably is chlorine.

In a subgroup of compounds of formula I (**compounds Ip**) R₄ is other than benzyl and R₅ is other than alkyl of 4 carbon atoms.

In a further subgroup of compounds of formula I R₁ is other than phenylalkyl. In a further subgroup R₉ is other than carboxy. In a further subgroup R₄, R₅ and R₆ are other than halogen in ortho position to the nitrogen moiety. In a further subgroup R₄, R₅, and R₆ are other than alkyl of more than 1 carbon atom. In a further subgroup R₁ is other than hydrogen. In a further subgroup R₃ is hydrogen. In a further subgroup, R₁ is propyl, isopropyl or 2-methylpropyl, R₃ and R₆ are hydrogen, R₄ is 5-halo and R₅ is 2-alkyl. In a further subgroup R₁ is alkyl of 8 to 22 carbon atoms.

Further subgroups of compounds of formula I are the compounds wherein
- R₁ is optionally substituted cycloalkyl as defined above;
- R₁ is cycloalkylalkyl or phenylalkyl as defined above;
- R₁ is -CR₇R₈R₉ or -CH₂CR₇R₈R₉ as defined above;
- R₁ is alkenyl as defined above, preferably of 5 to 22 carbon atoms;
- R₁ is alkadienyl or alkatrienyl as defined above; and/or
- R₃ is alkyl of 1 to 3 carbon atoms.

A further subgroup of compounds of formula I is the compounds of formula Is 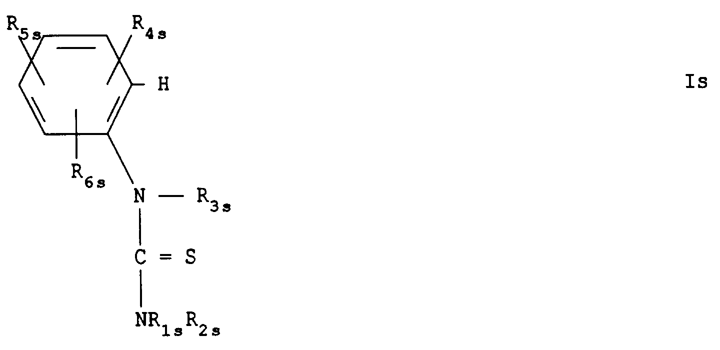
wherein
- R₁ₛ: is hydrogen; alkyl of 1 to 6 carbon atoms; cycloalkyl of 3 to 6 carbon atoms optionally monosubstituted by methyl; cyclopropylmethyl; phenylalkyl of 7 or 8 carbon atoms; hydroxyalkyl of 2 to 4 carbon atoms, methoxyethyl or mercaptoethyl with the hydroxy, methoxy or mercapto part attached in other than the 1-position; -CR₇ₛR₈ₛR₉ₛ or -CH₂CR₇ₛR₈ₛR₉ₛ wherein R₇ₛ and R₈ₛ either independently are hydrogen or methyl or together are unbranched alkylene of 3 to 5 carbon atoms, and R₉ₛ is carboxy, methoxycarbonyl or carbamoyl; alkenyl of 3 to 18 carbon atoms wherein the double bond is in other than the 1-position, or alkadienyl of 5 to 18 carbon atoms or alkatrienyl of 7 to 18 carbon atoms wherein each double bond is in other than the 1-position and no carbon atom is part of two double bonds, and
- R₂ₛ: is hydrogen;
- R₃ₛ: is hydrogen or methyl;
- R₄ₛ: is hydrogen; alkyl of 1 to 4 carbon atoms; methoxy; halogen of atomic number of from 9 to 35; trifluoromethyl; cyano; dimethylamino; nitro; phenyl; or benzyl;
- R₅ₛ: is hydrogen; alkyl of 1 to 4 carbon atoms; methoxy; halogen of atomic number 9 or 17; or trifluoromethyl; and
- R₆ₛ: is hydrogen; alkyl of 1 to 3 carbon atoms; or methoxy;
with the provisos that
i) when both R₄ₛ and R₅ₛ are trifluoromethyl, then they are not ortho to each other;
ii) when R₁ₛ is hydroxyalkyl and each of R₄ₛ and R₅ₛ is selected from hydrogen and alkyl,
   then at least two of R₄ₛ, R₅ₛ and R₆ₛ are alkyl; and
iii) when R₁ₛ is alkyl and R₄ₛ is phenyl,
   then R₅ₛ and R₆ₛ are other than methoxy;
   in free form or salt form as appropriate.

While many of the compounds of formula I are known per se, as well as their use in therapy, such as the compound of Example 60, from e.g. Rohm and Haas USP 3'950'537 as compound M therein, their use in the therapy of atherosclerosis is novel. Further background art is e.g. American Cyanamid GB-A-2113684 and Warner-Lambert EP-A-297610 disclosing structurally similar compounds with anti-atherosclerotic properties.

Further, for a subgroup of compounds of formula I no therapeutic use at all is known. The invention therefore also concerns a pharmaceutical composition comprising a compound of formula Ia 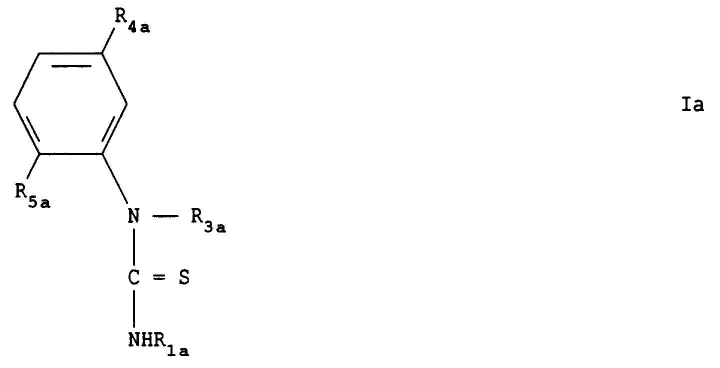
wherein
- R₁ₐ: is hydrogen; alkyl of 3 to 22 carbon atoms; cycloalkyl of 3 to 6 carbon atoms optionally mono- or independently disubstituted by alkyl of 1 or 2 carbon atoms; cycloalkylalkyl of 3 to 6 carbon atoms in the cycloalkyl and of 1 to 3 carbon atoms in the alkylene part; alkoxyalkyl or mercaptoalkyl of 2 to 4 carbon atoms in the alkylene part, of 1 to 3 carbon atoms in the alkoxy part, and with the alkoxy or mercapto part attached in other than the 1-position; -CR₇ₐR₈ₐR₉ₐ or -CH₂CR₇ₐR₈ₐR₉ₐ wherein R₇ₐ and R₈ₐ either independently are hydrogen, methyl or ethyl or together are unbranched alkylene of 3 to 5 carbon atoms, and R₉ₐ is alkoxycarbonyl of altogether 2 to 4 carbon atoms or carbamoyl; alkenyl of 4 to 22 carbon atoms wherein the double bond is in other than the 1-position, or alkadienyl of 5 to 22 carbon atoms or alkatrienyl of 7 to 22 carbon atoms wherein each double bond is in other than the 1-position and no carbon atom is part of two double bonds;
- R₃ₐ: is hydrogen or alkyl of 1 to 3 carbon atoms;
one of R₄ₐ and R₅ₐ is alkyl of 1 to 4 carbon atoms; alkoxy of 1 to 3 carbon atoms; halogen of atomic number of from 9 to 35; trifluoromethyl; cyano; dialkylamino of independently 1 to 3 carbon atoms in the alkyl parts; nitro; phenyl; or benzyl;
the other of R₄ₐ and R₅ₐ is alkyl of 1 to 4 carbon atoms; alkoxy of 1 to 3 carbon atoms; or trifluoromethyl;
with the provisos that
i) when both R₄ₐ and R₅ₐ are trifluoromethyl, then they are not ortho to each other; and
ii) when R₁ₐ is alkyl and one of R₄ₐ and R₅ₐ is phenyl, then the other of R₄ₐ and R₅ₐ is other than alkoxy;
   in free form or salt form as appropriate,
   together with a pharmaceutically acceptable carrier or diluent.

In a subgroup of compounds of formula Ia R₁ₐ is other than hydrogen. In a further subgroup R₄ₐ and R₅ₐ are other than both alkoxy. In a further subgroup R₁ₐ when it is branched alkyl of more than 3 carbon atoms is branched at a position other than at the carbon atom attached to the nitrogen atom. In a further subgroup R₁ₐ when it is alkyl is propyl or isopropyl or alkyl of 4 to 22 carbon atoms branched at a position other than at the carbon atom attached to the nitrogen atom. In a further subgroup R₁ₐ when it is alkyl is propyl, isopropyl or 2-methylpropyl. In a further subgroup R₄ₐ and R₅ₐ when they are alkyl are methyl; in a further subgroup they are other than benzyl.

A preferred group of compounds of formula Ia is the compounds of formula Ia' 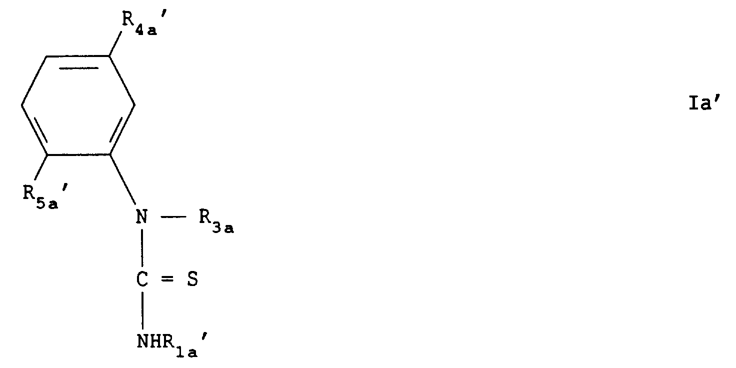
wherein
- R₁ₐ': is propyl or isopropyl or alkyl of 4 to 22 carbon atoms branched at a position other than at the carbon atom attached to the nitrogen atom; cycloalkyl of 3 to 6 carbon atoms optionally mono- or independently disubstituted by alkyl of 1 or 2 carbon atoms; cycloalkylalkyl of 3 to 6 carbon atoms in the cycloalkyl and of 1 to 3 carbon atoms in the alkylene part; alkoxyalkyl or mercaptoalkyl of 2 to 4 carbon atoms in the alkylene part, of 1 to 3 carbon atoms in the alkoxy part, and with the alkoxy or mercapto part attached in other than the 1-position; -CR₇ₐR₈ₐR₉ₐ or -CH₂CR₇ₐR₈ₐR₉ₐ wherein R₇ₐ, R₈ₐ and R₉ₐ are as defined above; alkenyl of 4 to 22 carbon atoms wherein the double bond is in other than the 1-position, or alkadienyl of 5 to 22 carbon atoms or alkatrienyl of 7 to 22 carbon atoms wherein each double bond is in other than the 1-position and no carbon atom is part of two double bonds;
- R₃ₐ: is as defined above;
one of R₄ₐ' and R₅ₐ' is methyl; alkoxy of 1 to 3 carbon atoms; halogen of atomic number of from 9 to 35; trifluoromethyl; cyano; dialkylamino of independently 1 to 3 carbon atoms in the alkyl parts; nitro; phenyl; or benzyl;
the other of R₄ₐ' and R₅ₐ' is methyl; alkoxy of 1 to 3 carbon atoms; or trifluoromethyl;
with the provisos that
i) when both R₄ₐ' and R₅ₐ' are trifluoromethyl, then they are not ortho to each other;
ii) when R₁ₐ' is alkyl and one of R₄ₐ' and R₅ₐ' is phenyl, then the other of R₄ₐ' and R₅ₐ' is other than alkoxy; and
iii)R₄ₐ' and R₅ₐ' are other than both alkoxy,
   in free form or salt form as appropriate,
   together with a pharmaceutically acceptable carrier or diluent.

In a subgroup of compounds of formula Ia' R₁ₐ' is propyl, isopropyl or 2-methylpropyl.

While some of the compounds of formula Ia are known per se, such as the compound of Example 29, from e.g. Hoechst USP 4'234'513 as Example 27 therein, and the compound of Example 30, from e.g. G.N. Vassilev and L.K. Iliev, Comptes Rendus Acad. bulg. Sci. 22 (5) (1969) 563-566 as compound 10. therein, their therapeutic use is novel.

Further, a particular subgroup of compounds of formula Ia is novel per se. The invention thus also concerns a compound of formula Iaa 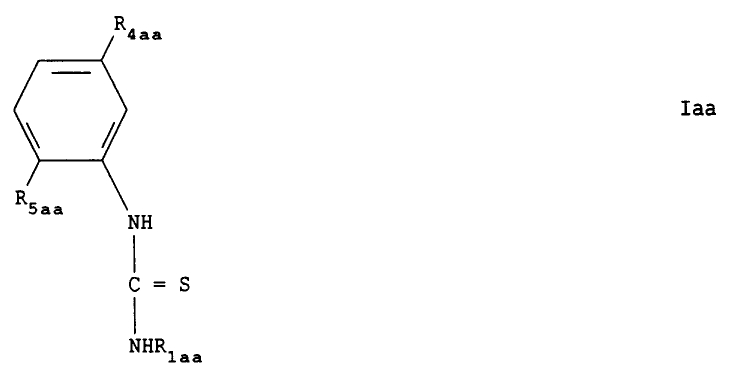
wherein
- R₁ₐₐ: is propyl, isopropyl or 2-methylpropyl;
- R₄ₐₐ: is halogen of atomic number of from 9 to 35; and
- R₅ₐₐ: is alkyl of 1 to 4 carbon atoms.

R₁ₐₐ preferably is propyl or 2-methylpropyl. R₄ₐₐ preferably is chlorine. R₅ₐₐ preferably is methyl, ethyl, isopropyl or t-butyl, preferably methyl, ethyl or isopropyl, especially methyl.

In a subgroup of compounds of formula Iaa R₁ₐₐ is propyl or 2-methylpropyl and R₅ₐₐ is alkyl of 1 to 3 carbon atoms; more particularly, R₁ₐₐ is propyl, R₄ₐₐ if fluoro or chloro and R₅ₐₐ is methyl or ethyl; even more particularly, R₅ₐₐ is methyl; even more particularly, R₁ₐₐ is 2-methylpropyl.

A part of the compounds of formula I is known and those which are not may be synthesized analogously to the known compounds of formula I. For example, a compound of formula I can be obtained by a process which comprises
a) for the preparation of a compound of formula I' 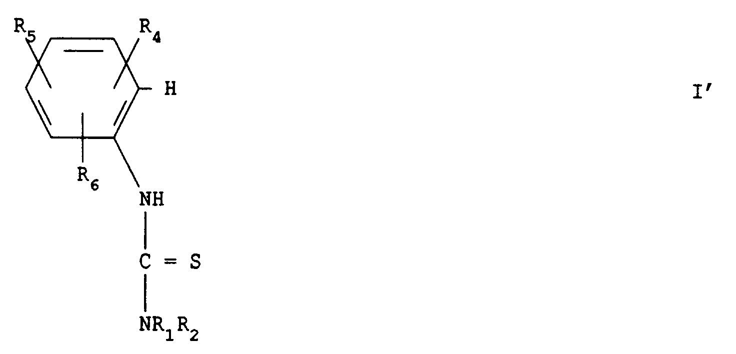 wherein the substituents are as defined above, reacting a corresponding compound of formula II 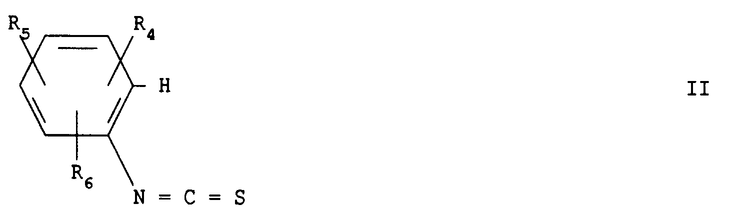 with a corresponding compound of formula III

   R₁R₂NH III

   or
b) for the preparation of a compound of formula I'' 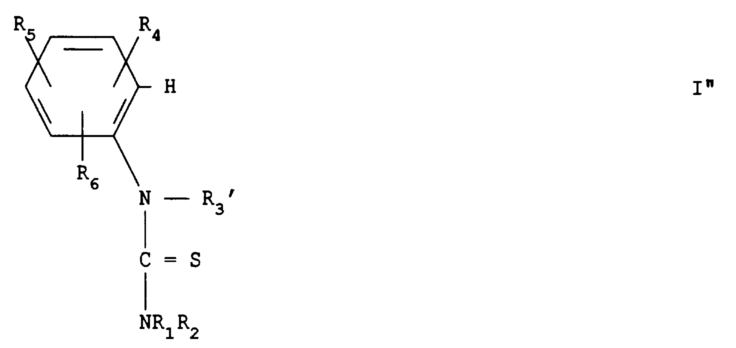 wherein R₃' is alkyl of 1 to 3 carbon atoms and
   the remaining substituents are as defined above, appropriately converting the oxo moiety to a thiono moiety in a corresponding compound of formula IV 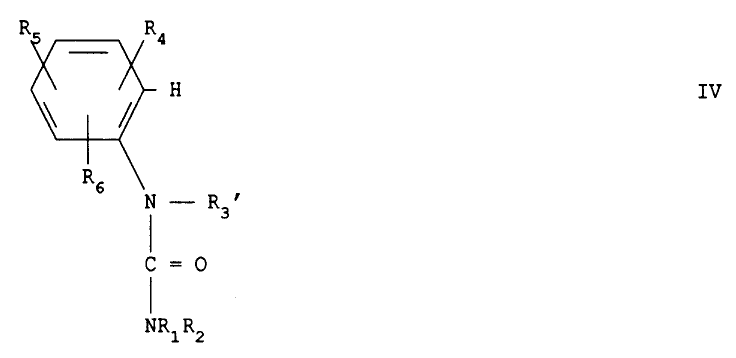 and recovering the resultant compound of formula I in free form or salt form as appropriate.

Thus a compound of formula Iaa can be obtained by a process which comprises reacting a corresponding compound of formula IIa 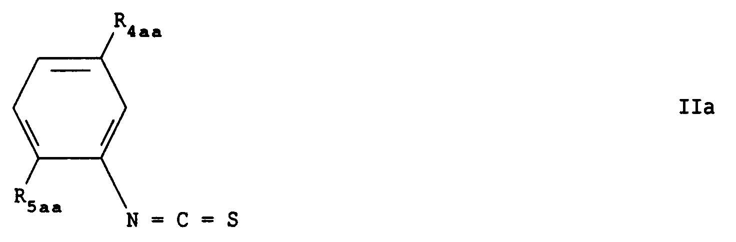
wherein R₄ₐₐ and R₅ₐₐ are as defined above, with a corresponding compound of formula IIIa

R₁ₐₐNH₂ IIIa

wherein R₁ₐₐ is as defined above.

The above process for the preparation of the compounds of formula I can be effected in conventional manner.

Process variant a) is a reaction of an isothiocyanate with an amine. The temperature preferably is from about 10° to about 40°C, preferably about 20° to about 30°C. An anhydrous inert organic solvent conveniently is used, such as a halogenated lower alkane, e.g. methylene chloride, or a C₁₋₃alkyl (C₂₋₃)alkanoate, e.g. ethyl acetate.

Process variant b) is a conversion of an urea to the corresponding thiourea. It preferably is effected with Lawessons's reagent (2,4-bis-4-methoxyphenyl-1,3-dithia-2,4-diphosphethane-2,4-disulfide). Preferably from about 0.5 to about 1 mole, especially from about 0.6 to about 0.85 mole Lawesson's reagent is used per mole compound of formula VI. The temperature preferably is from about 50°C to reflux temperature, preferably from about 100° to about 110°C, especially about 110°C. An anhydrous inert organic solvent conveniently is used, preferably a hydrocarbon such as benzene, toluene or xylene, especially toluene.

A compound of formula I can be isolated and, if desired, purified from the reaction mixture in conventional manner.

A compound in free form can be converted to a salt form where appropriate also in conventional manner, and vice-versa.

A compound having one or more centers of asymmetry may be obtained in optically pure form by using an optically pure starting material, or may be resolved into two optically active isomers by conventional techniques, e.g. via formation of a mixture of diastereoisomeric salts where salt formation is possible and subsequent separation by e.g. fractional crystallization or column chromatography.

Insofar as its preparation is not described herein, a compound used as a starting material is known or can be prepared by known methods starting from known compounds, e.g. as described in the Examples.

The following Examples illustrate the invention. All temperatures are in degrees Centigrade. MP= melting point; OR = optical rotation [α]_{D}²⁰ . The NMR spectra are taken at 200 MHz.

### Example 1: N-(5-Chloro-2-methylphenyl)-N'-propylthiourea

### [Formula I: R₁ = n-propyl; R₂, R₃, R₆ = H; R₄ = 5-chloro; R₅ = 2-methyl] [Formula Iaa: R₁ₐₐ = n-propyl; R₄ₐₐ = chloro; R₅ₐₐ = methyl] [Process variant a); reaction of an isothiocyanate with an amine]

163 g **n-propylamine** (compound of formula III) is added dropwise over a period of 40 minutes to a solution of 390 g of **5-chloro-2-methylphenylisothiocyanate** (compound of formula II) in 700 ml of ethyl acetate stirred at 15° under nitrogen at a rate such that the temperature is maintained at 25°-30°. On completion of the addition, the reaction mixture is stirred at 25°-30° for 5 minutes, 2.1 l of n-heptane is added, and the mixture is cooled to 0° over a period of 20 minutes. The resulting white suspension is stirred at 0° for 1 hour, and the solids are collected by vacuum filtration, washed with two 250 ml portions of cold (5°) n-heptane and dried at 25 torr and 40° for about 18 hours to constant weight. The **title compound** is obtained (white solid; MP 97°-98°).

### Example 2: N-(5-Chloro-2-methylphenyl)-N'-2-methylpropylthiourea

### [Formula I: R₁ = 2-methylpropyl; R₂, R₃, R₆ = H; R₄ = 5-chloro; R₅ = 2-methyl] [Formula Iaa: R₁ₐₐ = 2-methylpropyl; R₄ₐₐ = chloro; R₅ₐₐ = methyl] [Process variant a)]

A solution of 25.0 g of **5-chloro-2-methylphenylisothiocyanate** (compound of formula II) in 25 ml of methylene chloride is added dropwise to a solution of 10.0 g of **isobutylamine** (compound of formula III) in 150 ml of methylene chloride stirred at 20°-25°, and the reaction mixture is stirred at 20°-25° for 2 hours. The methylene chloride is distilled, and, as the distillation proceeds, methyl-t-butyl ether is gradually added. The resulting methyl t-butyl ether solution is allowed to cool to 20°-25°, and the resulting solid is collected by filtration, washed with methyl t-butyl ether and vacuum dried to constant weight. The **title compound** is obtained (MP 116.5°-117.5°).

### Example 3: N-(5-Chloro-2-methylphenyl)-N-methyl-N'-propylthiourea

### [Formula I: R₁ = n-propyl; R₂, R₆ = H; R₃ = methyl; R₄ = 5-chloro; R₅ = 2-methyl] [Formula Ia: R₁ₐ = n-propyl; R₃ₐ, R₅ₐ = methyl; R₄ₐ = chloro] [Process variant b); conversion]

A mixture of 1.8 g of crude **N-(5-chloro-2-methylphenyl)-N-methyl-N'-propylurea** (compound of formula IV) prepared as described hereunder and 2.5 g of Lawesson's reagent in 20 ml of toluene is stirred at 110° for 24 hours, the toluene is evaporated at reduced pressure, and the waxy solid residue is dissolved in the minimum amount of methylene chloride and flash chromatographed on a 230-400 mesh ASTM silica gel column. The fractions containing relatively pure product as determined by thin layer chromatography are combined and evaporated at reduced pressure to obtain an oil which crystallizes upon standing. The crystals are triturated with n-pentane to obtain the **title compound** (MP 83°-86°). A less pure second crop is similarly obtained from less pure fractions from the flash chromatography (MP 82°-84°).

The starting material is obtained as follows:
a) 1.5 g of **n-propylamine** is slowly added to a solution of 4.3 g of **5-chloro-2-methylphenylisocyanate** in 70 ml of methylene chloride stirred at 20°-25°, and, after the vigorous reaction subsides, the reaction mixture is stirred at 20°-25° for 10 minutes. The resulting solid is collected by filtration and washed with methylene chloride. **N-(5-chloro-2-methylphenyl)-N'-propylurea** is obtained (MP 182°-184°).
b) 600 mg of 60 % sodium hydride/mineral oil is washed with n-pentane, the sodium hydride is added to a solution of 2.7 g of **product from step a) above** in 25 ml of N,N-dimethylformamide stirred at 20°-25°, the mixture is stirred at 20°-25° for 1.5 hours, 1.8 g of **methyl iodide** is slowly added, and the reaction mixture is stirred at 20°-25° for 24 hours and poured into water. The mixture is extracted twice with methyl t-butyl ether, and the ether extracts are combined, dried over anhydrous sodium sulfate and evaporated at reduced pressure to an oil. The oil is fractionated on a Waters HPLC having a silica gel column using 60 % mixed hexanes/ethyl acetate as the eluant. **N-(5-chloro-2-methylphenyl)-N-methyl-N'-propylurea** is obtained (waxy solid).

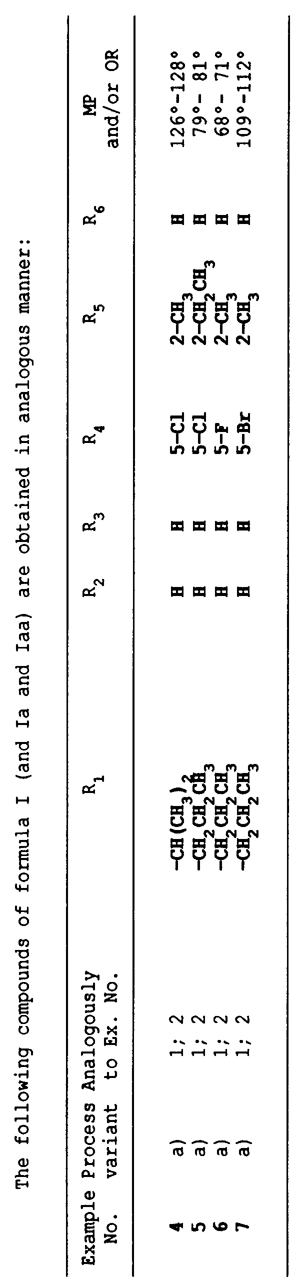

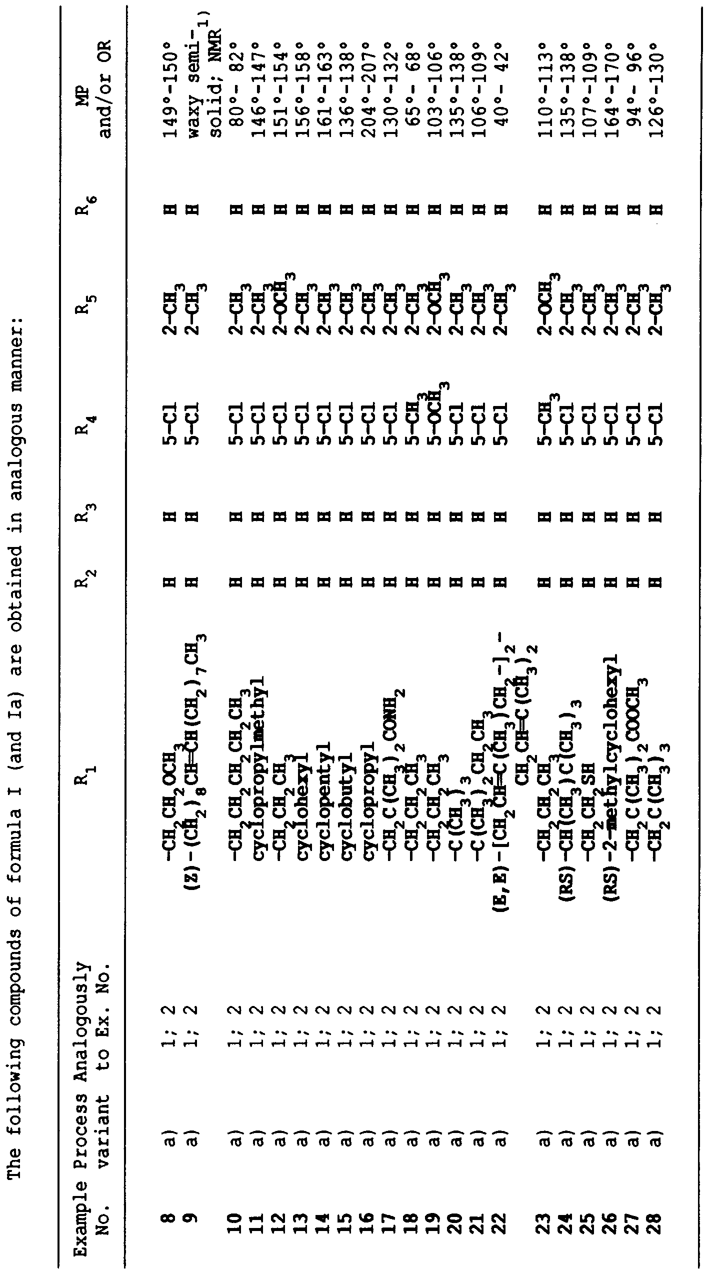

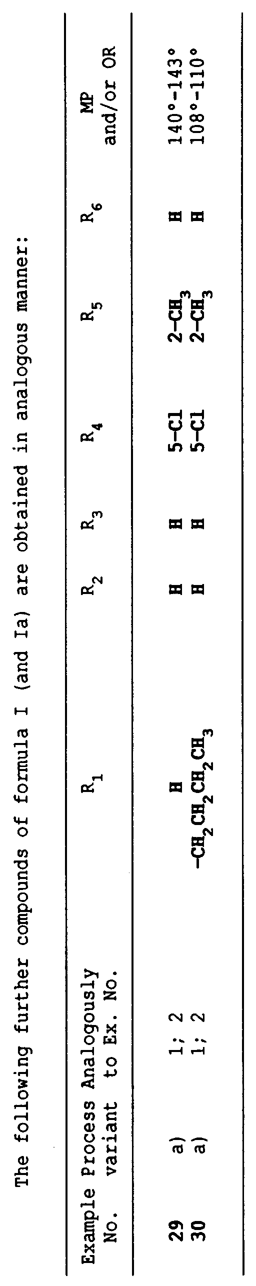

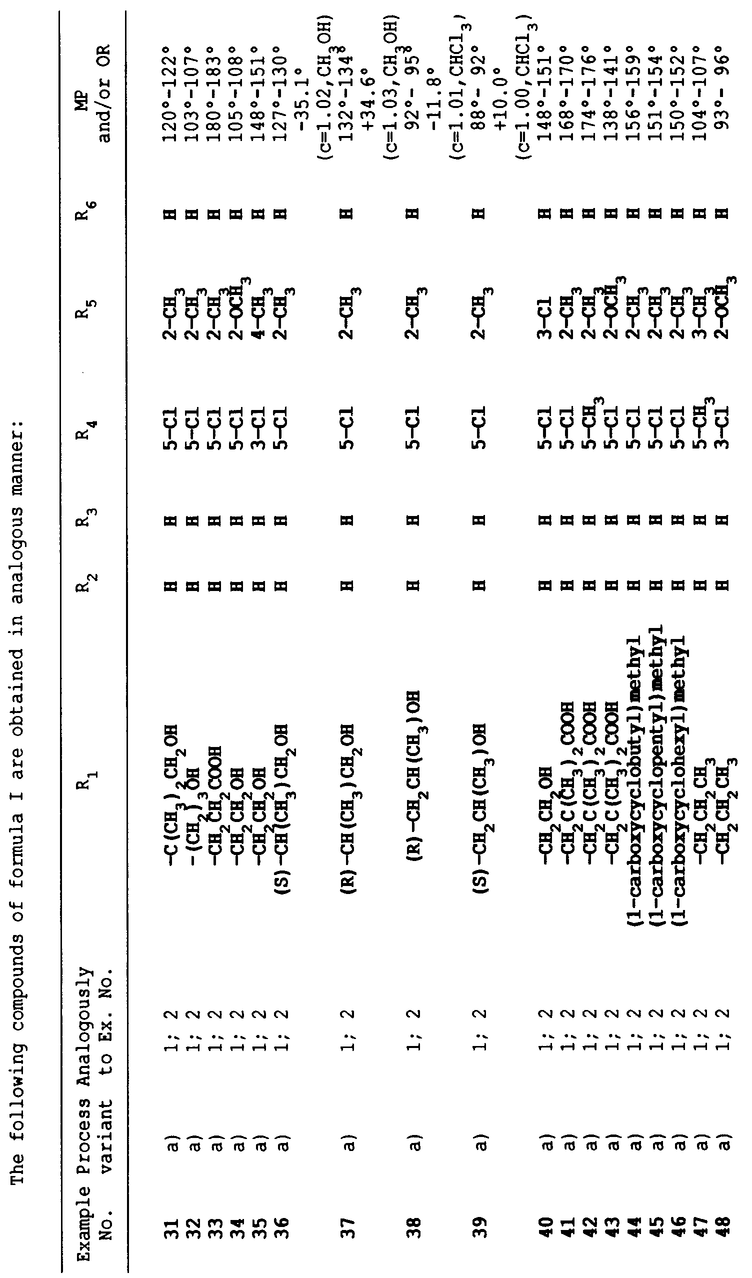

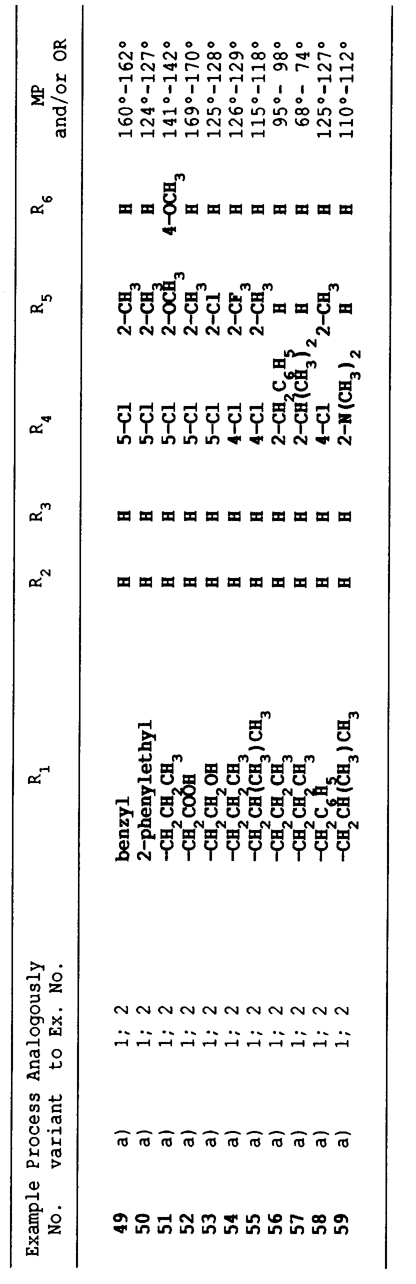

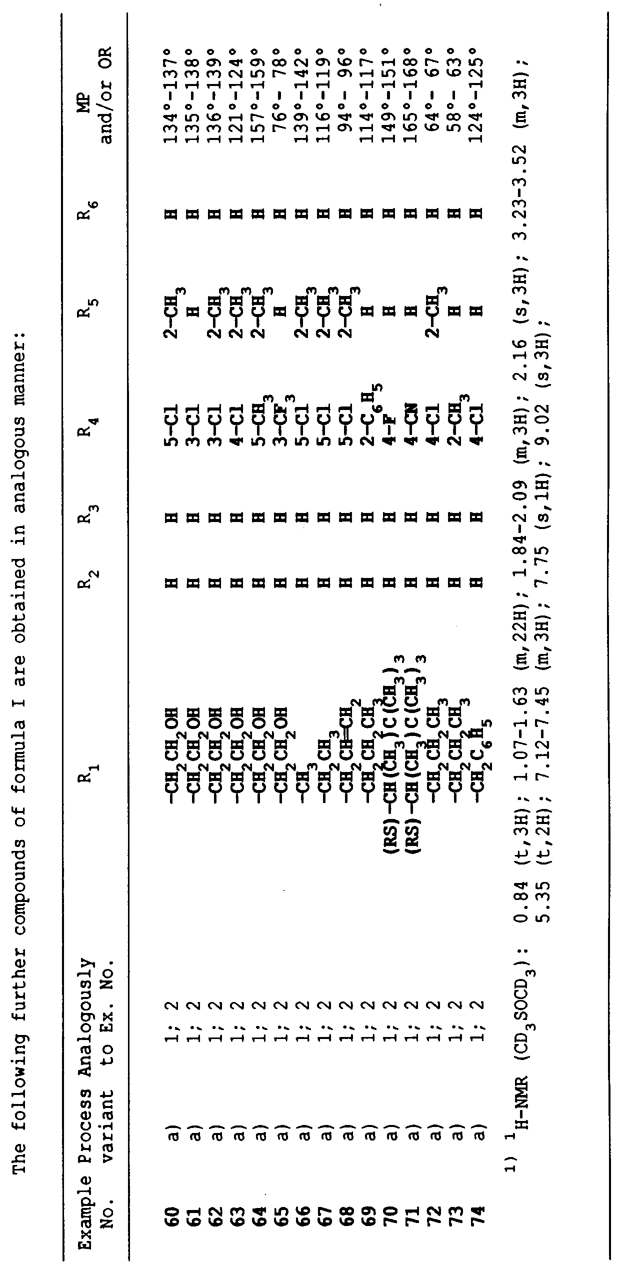

The compounds of formula I in free form or pharmaceutically acceptable salt form as appropriate, possess interesting pharmacological activity. They are indicated for use as pharmaceuticals.

In particular, they raise the blood serum high density lipoprotein [HDL; HDL-cholesterol and apolipoprotein A-I (Apo A-I)] level. Most of the compounds have the added benefit of lowering the blood serum total triglyceride level.

This activity can be determined in conventional assay methods, e.g. as follows:

### a) Test A: In vivo HDL-Cholesterol test:

Male Sprague-Dawley rats weighing 200-225 g are housed two per cage and fed Purina Rodent Chow Special Mix 5001-S supplemented with 0.25 % cholic acid and 0.75 % cholesterol by Purina and water ad libitum for seven or eight days. Each test substance is then administered to a group of six rats in the supplemented diet for eight or twenty-one days, the test substance constituing 0.005-0.20 % of the diet. Body weight and food consumption data are recorded prior to diet administration, prior to test substance administration and at termination. Typical doses of the test substance are 4-200 mg/kg/day.

At termination, the rats are anesthetized, sacrificed by exsanguination, the blood is collected, kept on ice while clotting, centrifuged, and the serum is separated. An aliquot is adjusted to a density of 1.06 g/ml with sodium chloride solution and refrigerated for overnight storage. Remaining sera aliquots are refrigerated and frozen for storage.

HDL is isolated by micro-ultracentrifugation (mUC) or fast protein liquid chromatography (FPLC) techniques. The mUC method is as follows: 175 µl of the 1.06 g/ml density-adjusted blood serum is centrifuged at 42000 rpm in a Beckmann 42.2 Ti rotor at 20°C for 2.5 hours. 95 µl is fractionated from the top, leaving 80 µl in the bottom. FPLC fractionation is performed by Superose^{R} 6 (highly cross-linked beaded agarose having a dry bead diameter of 20-40 µm) gel permeation chromatography using the method of Kieft et al., J.Lipid Res. 32 (1991) 859-866 modified by the technique described in France et al., Laboratory Robotics and Automation 2 (1990) 155-173. The column buffer is Tris buffered saline solution [i.e. 0.05 M Tris (2-amino-2-hydroxymethyl-1,3-propanediol) and 0.15 M sodium chloride in distilled deionized water (ddH₂O)] containing 0.01 % sodium azide. 200 µl of serum is injected and forty 0.5 ml fractions are collected.

Cholesterol is assayed on total, 42.2 Ti rotor bottoms and FPLC fractions using the Sigma Diagnostics kit for the enzymatic determination of cholesterol, Procedure No. 352, modified for use with ninety-six well microtiter plates. The reconstituted reagent (after addition of water) contains 300 U/l cholesterol oxidase, 100 U/l cholesterol esterase, 1000 U/l peroxidase (horseradish), 0.3 mmoles/l 4-aminoantipyrine and 30.0 mmoles/l p-hydroxybenzenesulfonate in a pH 6.5 buffer. This method uses cholesterol esterase to hydrolyze cholesterol esters to free cholesterol. Free cholesterol is oxidized to produce hydrogen peroxide that is used to form a quinoneimine dye. Since the reaction takes place quantitatively, the concentration of the dye, measured colorimetrically, is directly proportional to the cholesterol content of the sample. Calibrator, standard and sample may be diluted with saline if cholesterol concentrations cause readings outside the linear range. 20 µl of calibrator, standard or test sample is mixed with a 200 µl aliquot of reagent in a ninety-six well microtiter plate. Each mixture is incubated at 20°-25° for 25 minutes, and absorbance is determined by a colorimetric microtiter plate reader at 490, 492 or 500 nm.

Top cholesterol (i.e. LDL-cholesterol) is determined by subtraction. Quality of micro-ultracentrifugation separation is determined by Corning universal agarose gel electrophoresis with Fat Red 7B stain.

FPLC fraction Apo A-I content is assessed by the non-reducing SDS-PAGE method of France et al., J. Lipid Res. 30 (1989) 1997-2004. HDL-cholesterol is quantitated by assessing the cholesterol content of fractions containing Apo A-I and lacking immunoreactive Apo B protein.

Total triglycerides are assayed on blood serum using the Boehringer Mannheim Diagnostics Reagentset^{R} Triglycerides-GB kit (Cat. No. 877557) modified for microtiter plate assays as follows: The reagents are prepared according to the described method. 100 µl of Working Solution 1 and 20 µl of dilute blood serum [1:1 blood serum:saline (saline is 0.15 M sodium chloride in ddH₂O)] are added to each well of a ninety-six well microtiter plate, mixed and incubated at 20°-25° for at least 5 minutes. 100 µl of working solution 2 is added to each well, and the contents are mixed and incubated at 20°-25° for at least 5 minutes and read at 490, 492 or 500 nm. The total triglyceride content in mg/dl of blood serum is calculated by comparing the absorbance with the absorbance of known samples.

Other conventional methods may be used to assay the blood serum samples for HDL-cholesterol and total triglyceride content.

### b) Test B: Apo A-I test:

The Apo A-I blood serum level of the test animals of Test A is determined by rat Apo A-I Enzyme-Linked Immunosorbent Assay (ELISA) as follows:

### 1. Production and purification of rabbit anti-rat Apo A-I antibody:

Rat Apo A-I is purified from pooled rat blood serum by sequential ultracentrifugation and isolation of rat high-density lipoproteins (HDL). Following delipidation, Apo A-I is resolved from other HDL proteins by gel filtration chromatography. Antisera are raised in three rabbits (Pocono Rabbit Farms, Candensis, PA) by serial subcutaneous injections of purified rat Apo A-I by conventional immunization protocols. After one year of repeated bleedings, antisera are pooled, aliquoted and frozen at -20°. The crude antisera are defrosted, applied to a cyanogen bromide-activated Sepharose^{R} Cl-4B (beaded agarose having a 60-140 µm dry bead diameter) column to which purified human HDL had been covalently linked, the column is washed with saline, and the purified antibody is eluted with 1 M pH 3 glycine solution. The glycine is removed by dialysis utilizing Tris Buffered Saline Solution to obtain a concentrated solution of purified rabbit anti-rat Apo A-I antibody.

### 2. Buffers, reagents and standards:

- Sodium carbonate adsorption buffer: 2.25 g of sodium carbonate, 4.40 g of sodium bicarbonate and 0.15 g of sodium azide are dissolved in 1.4 l of ddH₂O, the pH is adjusted to 9.6 with sodium hydroxide, and the buffer is diluted with ddH₂O to a total volume of 1.5 l;
- pH 8 0.5 M Tris: a solution of 60.55 g of Tris·HCl (2-amino-2-hydroxymethyl-1,3-propanediol·hydrochloride) in 1.0 l of ddH₂O is adjusted to pH 8.0 with 10 N sodium hydroxide solution;
- secondary antibody buffer: 200 ml of pH 8 0.5 M Tris is added to a solution of 40 g of bovine serum albumin (BSA) Cohn Fraction V in 1.8 l of ddH₂O, 0.2 g of sodium azide is added, and 18.0 g of sodium chloride is added;
- substrate buffer: 0.1 g of sodium azide is added with stirring to 105.1 g of diethanolamine and 500 ml of ddH₂O, 10 ml of 0.5 M magnesium chloride·hexahydrate solution is added, the pH is adjusted to 9.8 with 12 N hydrochloric acid, and the solution is diluted to a total volume of 1 l with ddH₂O;
- 30 x ELISA wash buffer: 525.96 g of sodium chloride, 76.68 g of Tris, 6 g of sodium azide and 30 ml of Tween^{R}20 [polyoxyethylene (20) sorbitan monolaurate] are combined, the pH is adjusted to 7.3 with 12 N hydrochloric acid, and the solution is diluted to a total volume of 2 l with ddH₂O;
- 1 x ELISA wash buffer: 1 volume of 30 x ELISA wash buffer is diluted with 29 volumes of ddH₂O;
- sample diluent buffer: 886.34 ml of secondary antibody buffer and 113.66 ml of Tween^{R}20 are mixed well to obtain an 11.366 % v/v Tween^{R}20 solution;
- standard diluent buffers: (A) 780 ml of secondary antibody buffer and 220 ml of Tween^{R}20 are mixed well to obtain a 22 % v/v Tween^{R}20 solution. (B) 890 ml of secondary antibody buffer and 110 ml of Tween^{R}20 are mixed well to obtain an 11 % v/v Tween^{R}20 solution;
- rat serum pool A: this source of rat blood serum is used to construct the standard curve for each assay. It is calibrated against purified Apo A-I and contains 45 ± 2.7 mg Apo A-I/dl blood serum;
- rat serum pool B: this is used as a low internal standard in each assay for monitoring assay drift and contains 40 mg Apo A-I/dl blood serum;
- rat serum pool C: this is derived from rats treated with reference compound (gemfibrozil), is used as a high internal standard for monitoring assay drift and contains 70 mg Apo A-I/dl blood serum.

### 3. Procedure:

Purified rat Apo A-I from a frozen stock containing 942 µg/ml is diluted in sodium carbonate adsorption buffer to a final concentration of 3.7 µg/ml. Into each well of several Nunc polystyrene ninety-six well microtiter plates is added 100 µl of this solution. Apo A-I is allowed to adsorb onto the plates for 48 hours at 4°. Plates are washed with 1 x ELISA wash buffer, and non-specific sites on the plate are blocked by an overnight incubation of secondary antibody buffer (200 µl/well) also in the cold. Assay plates are stored in this state until used (for up to three weeks).

On the day of the assay, ELISA platees (2 plates, 36 rat serum samples per plate) are washed four times in 1 x ELISA wash buffer. 100 µl of 1 x ELISA wash buffer is left in each well until the test sample is introduced. 10 µl of each rat blood serum sample to be analyzed is mixed with 290 µl of sample diluent buffer for a final dilution of 1:30 and a final Tween^{R}20 concentration of 11 %. Sextuplicate samples of internal standard rat serum pools B and C are treated identically.

2.0 ml of rat serum pool A is mixed with an equal volume of standard diluent buffer A to yield a dilution of 1:2 and a Tween^{R}20 concentration of 11 %. The sample is then serially diluted in standard diluent buffer B to yield dilutions of 1:8, 1:16, 1:32, 1:64 and 1:128.

Diluted standards and samples in non-ELISA microtiter plates are placed into a heated incubator at 52° for 2 hours. After cooling to 20°-25°, wash buffer is removed from ELISA test plates. 75 µl of standards and test samples are transferred to ELISA test plates. 75 µl of purified rabbit anti-rat Apo A-I antibody solution, diluted 1:75 in secondary antibody buffer, is then added to each well. Each plate is shaken briefly to assure mixing of antibody and sample, sealed and allowed to incubate for 18 hours at 20°-25°. After this incubation, each plate is washed in 1 x ELISA wash buffer four times. To each well is then added 100 µl of an alkaline phosphatase conjugated goat anti-rabbit IgG antibody, diluted 1:1000 in secondary antibody buffer. This is allowed to interact for 3 hours at 20°-25°. Each plate is washed again and aspirated dry, and to each well is added substrate buffer containing 1 mg/ml disodium p-nitrophenylphosphate, a chromogenic substrate which yields a color finversely proportional to the amount of Apo A-I in the sample. Each plate is monitored in an ELISA spectrophotometer for 1 to 3 hours. When wells which received no Apo A-I (maximum color) reach an optical density of 1.0 at 405 nm, each plate is read by the ELISA reader.

The standard curve is plotted as optical density versus log of the concentration. The unknowns are related to this curve, and the Apo A-I content is expressed in mg/dl.

The above tests A and B indicate that the compounds are active at a dosage in the range of from about 10 mg/kg to about 200 mg/kg per day.

The compounds are therefore indicated for use in raising the blood serum high density lipoprotein (HDL; HDL-cholesterol and apolipoprotein A-I) level and many of them also for lowering the blood serum total triglyceride level and thus in the treatment of atherosclerosis.

The precise dosage to be employed depends of course upon several factors including the host, the nature and the severity of the condition being treated, the mode of administration and the particular active substance employed. However, in general, satisfactory results are indicated to be obtained at daily dosages in the range of from about 400 mg to about 2000 mg, conveniently administered in divided doses two to four times a day.

The compounds may be administered by any conventional route, in particular enterally, preferably orally e.g. in the form of tablets or capsules, or parenterally e.g. in the form of sterile injectable solutions or suspensions.

The compounds of Examples 1, 2, 4, 56 and 57 are preferred, particularly those of Examples 1, 2 and 56, especially the compound of Example 2.

Thus e.g. the following activity has been determined:

| Compound | Dose (mg/kg/day) | Duration of treatment (days) | HDL-cholesterol level increase (%) | Apo-I level increase (%) | Total triglycerides level decrease (%) |
|---|---|---|---|---|---|
| Example 1 | 60 | 8 | 225 | 65 | 45 |
| Example 2 | 79 | 8 | 267 | 69 | 49 |
| Gemfibrozil | 50 | 8 | 100 | 50 | 35 |
| Example 1 | 65 | 21 | 256 | 47 | 70 |
| Example 2 | 73 | 21 | 323 | 102 | 44 |
| Gemfibrozil | 44 | 21 | 106 | 44 | 43 |

It is therefore indicated that, for this use, the compounds of Examples 1 and 2 may be administered at dosages lower than those conventionally employed with gemfibrozil by similar modes of administration, i.e. about 400-1000 mg/day orally.

Pharmaceutical compositions comprising a compound of formula I in free form or pharmaceutically acceptable salt form as appropriate together with at least one pharmaceutically acceptable carrier or diluent, may be manufactured in conventional manner. They may be e.g. in unit dosage form containing, for example, from about 100 mg to about 500 mg of active compound.

The invention thus concerns a process for the preparation of a medicament against atherosclerosis which comprises mixing a compound of formula I in free form or salt form as appropriate with at least one pharmaceutically acceptable carrier or diluent.

It further comprises the use of a compound of formula I in free form or salt form as appropriate for the manufacture of a medicament against atherosclerosis.

The invention also concerns a pharmaceutical composition comprising a compound of formula Ia in free form or pharmaceutically acceptable salt form as appropriate, together with at least one pharmaceutically acceptable carrier or diluent.

It further comprises a compound of formula Ia in free form or pharmaceutically acceptable salt form as appropriate, for use as a pharmaceutical, particularly for use in the prophylactic or curative treatment of atherosclerosis.

## Claims (Claims for the following Contracting State(s): AT, BE, CH, LI, DE, DK, FR, GB, IT, LU, NL, PT, SE)

1. Use of a compound of formula I 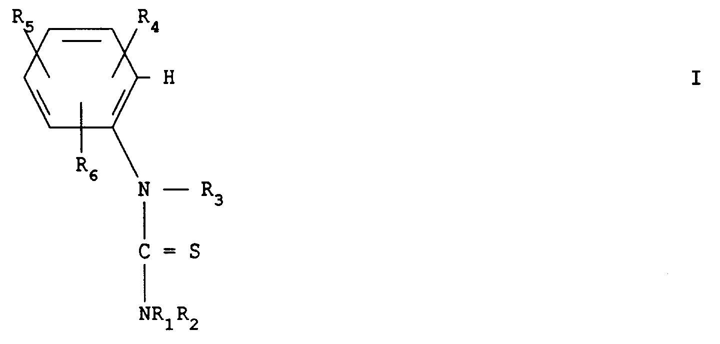 wherein
R₁ is hydrogen; alkyl of 1 to 22 carbon atoms; cycloalkyl of 3 to 6 carbon atoms optionally mono- or independently disubstituted by alkyl of 1 or 2 carbon atoms; cycloalkylalkyl of 3 to 6 carbon atoms in the cycloalkyl and of 1 to 3 carbon atoms in the alkylene part or phenylalkyl of altogether 7 to 9 carbon atoms; hydroxyalkyl, alkoxyalkyl or mercaptoalkyl of 2 to 4 carbon atoms in the alkylene part, of 1 to 3 carbon atoms in the alkoxy part, and with the hydroxy, alkoxy or mercapto part attached in other than the 1-position; -CR₇R₈R₉ or -CH₂CR₇R₈R₉ wherein R₇ and R₈ either independently are hydrogen, methyl or ethyl or together are unbranched alkylene of 3 to 5 carbon atoms, and R₉ is carboxy, alkoxycarbonyl of altogether 2 to 4 carbon atoms or carbamoyl; alkenyl of 3 to 22 carbon atoms wherein the double bond is in other than the 1-position, or alkadienyl of 5 to 22 carbon atoms or alkatrienyl of 7 to 22 carbon atoms wherein each double bond is in other than the 1-position and no carbon atom is part of two double bonds, and
R₂ is hydrogen;
R₃ is hydrogen or alkyl of 1 to 3 carbon atoms;
R₄ is hydrogen; alkyl of 1 to 4 carbon atoms; alkoxy of 1 to 3 carbon atoms; halogen of atomic number of from 9 to 35; trifluoromethyl; cyano; dialkylamino of independently 1 to 3 carbon atoms in the alkyl parts; nitro; phenyl; or benzyl;
R₅ is hydrogen; alkyl of 1 to 4 carbon atoms; alkoxy of 1 to 3 carbon atoms; halogen of atomic number 9 or 17; or trifluoromethyl; and
R₆ is hydrogen; alkyl of 1 to 3 carbon atoms; or alkoxy of 1 to 3 carbon atoms;
with the provisos that
i) when both R₄ and R₅ are trifluoromethyl, then they are not ortho to each other;
ii) when R₁ is hydroxyalkyl and each of R₄, R₅ and R₆ is selected from hydrogen and alkyl, then at least two of R₄, R₅ and R₆ are alkyl; and
iii) when R₁ is alkyl and R₄ is phenyl,
then R₅ and R₆ are other than alkoxy;
in free form or salt form as appropriate,
for the manufacture of a medicament against atherosclerosis or of a medicament for raising blood serum high density lipoprotein level.

2. A process for the preparation of a medicament against atherosclerosis or of a medicament for raising blood serum high density lipoprotein level which comprises mixing a compound of formula I as defined in claim 1 in free form or salt form as appropriate with at least one pharmaceutically acceptable carrier or diluent.

3. Use according to claim 1 or process according to claim 2 wherein the compound of formula I is as defined in claim 1 with the further proviso that R₄ is other than benzyl and R₅ is other than alkyl of 4 carbon atoms.

4. Use according to claim 1 or process according to claim 2 wherein the compound of formula I is a compound of formula Is 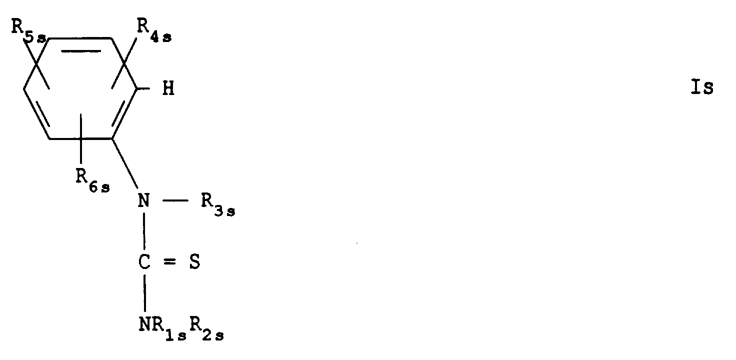 wherein
R₁ₛ is hydrogen; alkyl of 1 to 6 carbon atoms; cycloalkyl of 3 to 6 carbon atoms optionally monosubstituted by methyl; cyclopropylmethyl; phenylalkyl of 7 or 8 carbon atoms; hydroxyalkyl of 2 to 4 carbon atoms, methoxyethyl or mercaptoethyl with the hydroxy, methoxy or mercapto part attached in other than the 1-position; -CR₇ₛR₈ₛR₉ₛ or -CH₂CR₇ₛR₈ₛR₉ₛ wherein R₇ₛ and R₈ₛ either independently are hydrogen or methyl or together are unbranched alkylene of 3 to 5 carbon atoms, and R₉ₛ is carboxy, methoxycarbonyl or carbamoyl; alkenyl of 3 to 18 carbon atoms wherein the double bond is in other than the 1-position, or alkadienyl of 5 to 18 carbon atoms or alkatrienyl of 7 to 18 carbon atoms wherein each double bond is in other than the 1-position and no carbon atom is part of two double bonds, and
R₂ₛ is hydrogen;
R₃ₛ is hydrogen or methyl;
R₄ₛ is hydrogen; alkyl of 1 to 4 carbon atoms; methoxy; halogen of atomic number of from 9 to 35; trifluoromethyl; cyano; dimethylamino; nitro; phenyl; or benzyl;
R₅ₛ is hydrogen; alkyl of 1 to 4 carbon atoms; methoxy; halogen of atomic number 9 or 17; or trifluoromethyl; and
R₆ₛ is hydrogen; alkyl of 1 to 3 carbon atoms; or methoxy;
with the provisos that
i) when both R₄ₛ and R₅ₛ are trifluoromethyl, then they are not ortho to each other;
ii) when R₁ₛ is hydroxyalkyl and each of R₄ₛ and R₅ₛ is selected from hydrogen and alkyl,
then at least two of R₄ₛ, R₅ₛ and R₆ₛ are alkyl; and
iii) when R₁ₛ is alkyl and R₄ₛ is phenyl,
then R₅ₛ and R₆ₛ are other than methoxy;
in free form or salt form as appropriate.

5. A pharmaceutical composition comprising a compound of formula I as defined in claim 1 which is of formula Ia 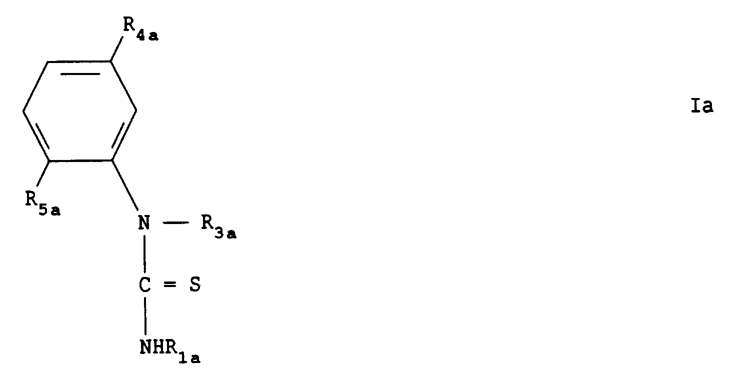 wherein
R₁ₐ is hydrogen; alkyl of 3 to 22 carbon atoms; cycloalkyl of 3 to 6 carbon atoms optionally mono- or independently disubstituted by alkyl of 1 or 2 carbon atoms; cycloalkylalkyl of 3 to 6 carbon atoms in the cycloalkyl and of 1 to 3 carbon atoms in the alkylene part; alkoxyalkyl or mercaptoalkyl of 2 to 4 carbon atoms in the alkylene part, of 1 to 3 carbon atoms in the alkoxy part, and with the alkoxy or mercapto part attached in other than the 1-position; -CR₇ₐR₈ₐR₉ₐ or -CH₂CR₇ₐR₈ₐR₉ₐ wherein R₇ₐ and R₈ₐ either independently are hydrogen, methyl or ethyl or together are unbranched alkylene of 3 to 5 carbon atoms, and R₉ₐ is alkoxycarbonyl of altogether 2 to 4 carbon atoms or carbamoyl; alkenyl of 4 to 22 carbon atoms wherein the double bond is in other than the 1-position, or alkadienyl of 5 to 22 carbon atoms or alkatrienyl of 7 to 22 carbon atoms wherein each double bond is in other than the 1-position and no carbon atom is part of two double bonds;
R₃ₐ is hydrogen or alkyl of 1 to 3 carbon atoms;
one of R₄ₐ and R₅ₐ is alkyl of 1 to 4 carbon atoms; alkoxy of 1 to 3 carbon atoms; halogen of atomic number of from 9 to 35; trifluoromethyl; cyano; dialkylamino of independently 1 to 3 carbon atoms in the alkyl parts; nitro; phenyl; or benzyl;
the other of R₄ₐ and R₅ₐ is alkyl of 1 to 4 carbon atoms; alkoxy of 1 to 3 carbon atoms; or trifluoromethyl;
with the provisos that
i) when both R₄ₐ and R₅ₐ are trifluoromethyl, then they are not ortho to each other; and
ii) when R₁ₐ is alkyl and one of R₄ₐ and R₅ₐ is phenyl, then the other of R₄ₐ and R₅ₐ is other than alkoxy;
in free form or salt form as appropriate,
together with a pharmaceutically acceptable carrier or diluent.

6. A pharmaceutical composition according to claim 5 wherein the compound of formula Ia is of formula Ia' 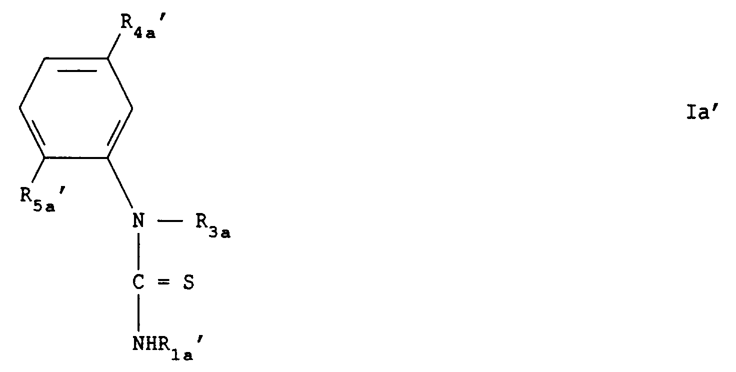 wherein
R₁ₐ' is propyl or isopropyl or alkyl of 4 to 22 carbon atoms branched at a position other than at the carbon atom attached to the nitrogen atom; cycloalkyl of 3 to 6 carbon atoms optionally mono- or independently disubstituted by alkyl of 1 or 2 carbon atoms; cycloalkylalkyl of 3 to 6 carbon atoms in the cycloalkyl and of 1 to 3 carbon atoms in the alkylene part; alkoxyalkyl or mercaptoalkyl of 2 to 4 carbon atoms in the alkylene part, of 1 to 3 carbon atoms in the alkoxy part, and with the alkoxy or mercapto part attached in other than the 1-position; -CR₇ₐR₈ₐR₉ₐ or -CH₂CR₇ₐR₈ₐR₉ₐ wherein R₇ₐ, R₈ₐ and R₉ₐ are as defined above; alkenyl of 4 to 22 carbon atoms wherein the double bond is in other than the 1-position, or alkadienyl of 5 to 22 carbon atoms or alkatrienyl of 7 to 22 carbon atoms wherein each double bond is in other than the 1-position and no carbon atom is part of two double bonds;
R₃ₐ is as defined in claim 5;
one of R₄ₐ' and R₅ₐ' is methyl; alkoxy of 1 to 3 carbon atoms; halogen of atomic number of from 9 to 35; trifluoromethyl; cyano; dialkylamino of independently 1 to 3 carbon atoms in the alkyl parts; nitro; phenyl; or benzyl;
the other of R₄ₐ' and R₅ₐ' is methyl; alkoxy of 1 to 3 carbon atoms; or trifluoromethyl;
with the provisos that
i) when both R₄ₐ' and R₅ₐ' are trifluoromethyl,
then they are not ortho to each other;
ii) when R₁ₐ' is alkyl and one of R₄ₐ' and R₅ₐ' is phenyl,
then the other of R₄ₐ' and R₅ₐ' is other than alkoxy; and
iii)R₄ₐ' and R₅ₐ' are other than both alkoxy.

7. A compound of formula I as defined in claim 1 which is of formula Iaa 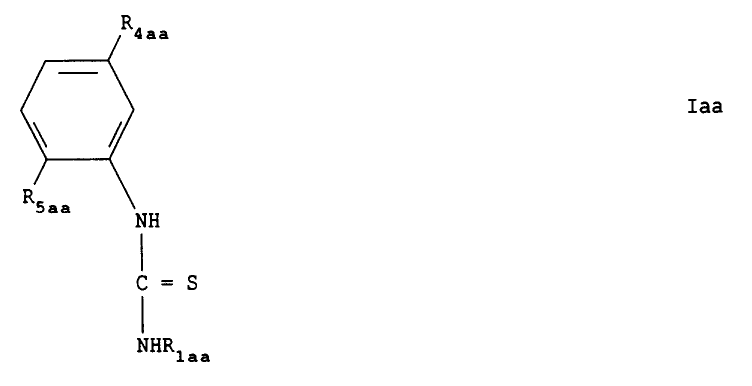 wherein
R₁ₐₐ is propyl, isopropyl or 2-methylpropyl;
R₄ₐₐ is halogen of atomic number of from 9 to 35; and
R₅ₐₐ is alkyl of 1 to 4 carbon atoms.

8. The compound of formula Iaa according to claim 7 which is N-(5-chloro-2-methylphenyl)-N'-propylthiourea.

9. The compound of formula Iaa according to claim 7 which is N-(5-chloro-2-methylphenyl)-N'-2-methylpropylthiourea.

10. The compound of formula Iaa according to claim 7 wherein R₁ₐₐ, R₄ₐₐ an*d* R₅ₐₐ are, respectively, either
- CH(CH₃)₂, 5-Cl and 2-CH₃, or
- CH₂CH₂CH₃, 5-Cl and 2-CH₂CH₃, or
- CH₂CH₂CH₃, 5-F and 2-CH₃, or
- CH₂CH₂CH₃, 5-Br and 2-CH₃.

11. A compound of formula Ia as defined in claim 5 for use as a pharmaceutical.

12. A compound according to claim 11 for use in the prophylactic or curative treatment of atherosclerosis or for raising blood serum high density lipoprotein level.

13. A process for the preparation of a compound of formula Iaa as defined in claim 7 which comprises reacting a corresponding compound of formula IIa 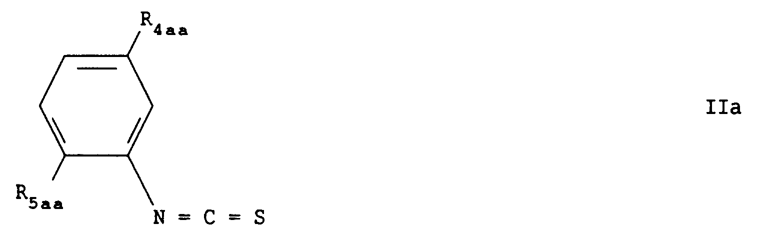 with a corresponding compound of formula IIIa
R₁ₐₐNH₂ IIIa
and recovering the resultant compound of formula I in free form or salt form as appropriate.

## Claims (Claims for the following Contracting State(s): ES, GR)

1. A process for the preparation of a compound of formula Iaa 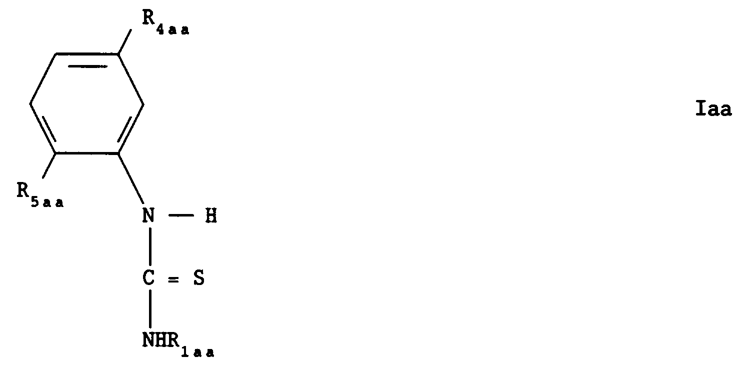 wherein
R₁ₐₐ is propyl, isopropyl or 2-methylpropyl;
R₄ₐₐ is halogen of atomic number of from 9 to 35; and
R₅ₐₐ is alkyl of 1 to 4 carbon atoms;
in free form or salt form as appropriate,
which comprises reacting a corresponding compound of formula IIa 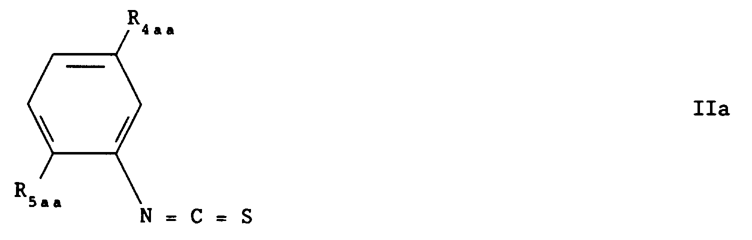 with a corresponding compound of formula IIIa
R₁ₐₐNH₂ IIIa
and recovering the resultant compound of formula I in free form or salt form as appropriate.

2. A process according to claim 1 for the preparation of the compound of formula Iaa which is N-(5-chloro-2-methylphenyl)-N'-propylthiourea.

3. A process according to claim 1 for the preparation of the compound of formula Iaa which is N-(5-chloro-2-methylphenyl)-N'-2-methylpropylthiourea.

4. A process according to claim 1 for the preparation of the compound of formula Iaa wherein R₁ₐₐ, R₄ₐₐ and R₅ₐₐ are, respectively, either
- CH(CH₃)₂, 5-Cl and 2-CH₃, or
- CH₂CH₂CH₃, 5-Cl and 2-CH₂CH₃, or
- CH₂CH₂CH₃, 5-F and 2-CH₃, or
- CH₂CH₂CH₃, 5-Br and 2-CH₃.

5. A process for the preparation of a medicament against atherosclerosis or of a medicament for raising blood serum high density lipoprotein level which comprises mixing a compound of formula Iaa as defined in claim 1 in free form or salt form as appropriate with at least one pharmaceutically acceptable carrier or diluent.

## Patentansprüche (Patentansprüche für folgende(n) Vertragsstaat(en): AT, BE, CH, LI, DE, DK, FR, GB, IT, LU, NL, PT, SE)

1. Verwendung einer Verbindung der Formel I 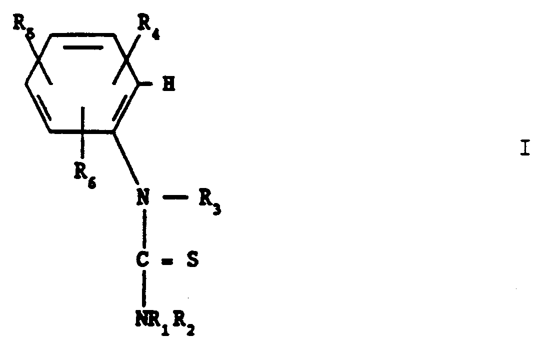 worin
R₁ Wasserstoff; ein Alkylrest mit 1 bis 22 Kohlenstoffatomen; ein Cycloalkylrest mit 3 bis 6 Kohlenstoffatomen, der gegebenenfalls mit Alkylresten mit 1 bis 2 Kohlenstoffatomen mono- oder unabhängig disubstituiert ist; ein Cycloalkylalkylrest mit 3 bis 6 Kohlenstoffatomen in dem Cycloalkylanteil und 1 bis 3 Kohlenstoffatomen in dem Alkylenteil oder ein Phenylalkylrest mit insgesamt 7 bis 9 Kohlenstoffatomen; ein Hydroxyalkyl-, Alkoxyalkyl- oder Mercaptoalkylrest mit 2 bis 4 Kohlenstoffatomen im Alkylenteil, 1 bis 3 Kohlenstoffatomen im Alkoxyteil, wobei der Hydroxy-, Alkoxy- oder Mercaptoteil an einer anderen Position als Position 1 gebunden ist; -CR₇R₈R₉ oder -CH₂CR₇R₈R₉, worin R₇ und R₈ entweder unabhängig Wasserstoff, Methyl- oder Ethylreste sind oder zusammen einen unverzweigten Alkylenrest mit 3 bis 5 Kohlenstoffatomen bilden und R₉ ein Carboxy-, ein Alkoxycarbonylmit insgesamt 2 bis 4 Kohlenstoffatomen oder Carbamoylrest ist; ein Alkenylrest mit 3 bis 22 Kohlenstoffatomen, worin die Doppelbindung an einer anderen Position als Position 1 ist oder ein Alkadienylrest mit 5 bis 22 Kohlenstoffatomen oder ein Alkatrienylrest mit 7 bis 22 Kohlenstoffatomen ist, worin jede Doppelbindung an einer anderen Position als Position 1 ist und kein Kohlenstoffatom Teil von 2 Doppelbindungen ist und
R₂ Wasserstoff ist;
R₃ Wasserstoff oder ein Alkylrest mit 1 bis 3 Kohlenstoffatomen ist;
R₄ Wasserstoff; ein Alkylrest mit 1 bis 4 Kohlenstoffatomen; ein Alkoxyrest mit 1 bis 3 Kohlenstoffatomen; Halogen mit einer Atomzahl von 9 bis 35; ein Trifluormethylrest; Cyanorest; Dialkylaminorest mit unabhängig 1 bis 3 Kohlenstoffatomen in den Alkylanteilen; ein Nitrorest; Phenylrest oder Benzylrest ist;
R₅ Wasserstoff; ein Alkylrest mit 1 bis 4 Kohlenstoffatomen; ein Alkoxyrest mit 1 bis 3 Kohlenstoffatomen; ein Halogen mit einer Atomzahl von 9 oder 17; oder ein Trifluormethylrest ist und
R₆ Wasserstoff; ein Alkylrest mit 1 bis 3 Kohlenstoffatomen oder ein Alkoxyrest mit 1 bis 3 Kohlenstoffatomen ist
mit dem Vorbehalt, daß dann, wenn
i) beide Reste R₄ und R₅ Trifluormethylreste sind, daß sie dann nicht in ortho-Stellung zueinander sind;
ii) wenn R₁ ein Hydroxyalkylrest ist und jeder der Reste R₄, R₅ und R₆ ausgewählt ist aus Wasserstoff und Alkylresten, daß dann mindestens zwei Reste der Reste R₄, R₅ und R₆ Alkylreste sind und
iii) wenn R₁ ein Alkylrest ist und R₄ ein Phenylrest ist, R₅ und R₆ etwas anderes als Alkoxyreste sind;
je nachdem in freier Form oder Salzform, zur Herstellung eines Arzneimittels gegen Atherosklerose oder eines Arzneimittels zur Erhöhung des Blutserumgehaltes an hochdichtem Lipoprotein.

2. Verfahren zur Herstellung eines Arzneimittels gegen Atherosklerose oder eines Arzneimittels zur Erhöhung des Blutserumgehaltes an hochdichtem Lipoprotein umfassend, daß man eine Verbindung der Formel I, wie in Anspruch 1 definiert, je nachdem in freier Form oder Salzform, mit mindestens einem pharmazeutisch annehmbaren Träger oder Verdünnungsmittel vermischt.

3. Verwendung nach Anspruch 1 oder Verfahren nach Anspruch 2, worin die Verbindung der Formel I wie in Anspruch 1 definiert ist, mit dem weiteren Vorbehalt, daß R₄ etwas anderes als ein Benzylrest und R₅ etwas anderes als ein Alkylrest mit 4 Kohlenstoffatomen ist.

4. Verwendung nach Anspruch 1 oder Verfahren nach Anspruch 2, worin die Verbindung der Formel I eine Verbindung der Formel Is 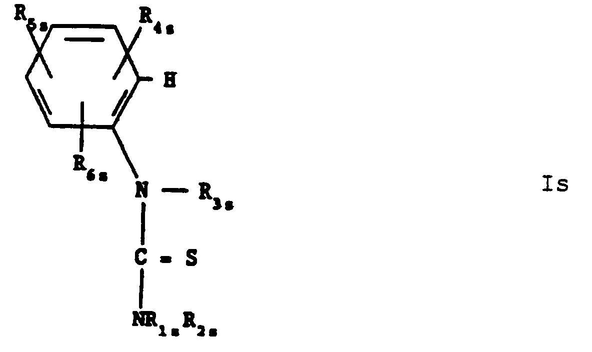 ist, worin
R₁ₛ Wasserstoff; ein Alkylrest mit 1 bis 6 Kohlenstoffatomen; ein Cycloalkylrest mit 3 bis 6 Kohlenstoffatomen, der gegebenenfalls mit einem Methylrest monosubstituiert ist; ein Cyclopropylmethylrest; ein Phenylalkylrest mit 7 oder 8 Kohlenstoffatomen; ein Hydroxyalkylrest mit 2 bis 4 Kohlenstoffatomen, ein Methoxyethyl- oder Mercaptoethylrest, wobei die Hydroxy-, Methoxy- oder Mercaptoanteile an einer anderen Position als Position 1 gebunden sind; -CR₇ₛR₈ₛR₉ₛ oder -CH₂CR₇ₛR₈ₛR₉ₛ, worin R₇ₛ und R₈ₛ entweder unabhängig Wasserstoff oder Methylreste sind oder zusammen einen unverzweigten Alkylenrest mit 3 bis 5 Kohlenstoffatomen bilden und R₉ₛ ein Carboxy-, Methoxycarbonyl- oder Carbamoylrest ist; ein Alkenylrest mit 3 bis 18 Kohlenstoffatomen, bei dem die Doppelbindung an einer anderen Position als Position 1 ist, oder ein Alkadienylrest mit 5 bis 18 Kohlenstoffatomen oder ein Alkatrienylrest mit 7 bis 18 Kohlenstoffatomen ist, worin jede Doppelbindung an anderer Position als Position 1 ist und kein Kohlenstoffatom Teil von 2 Doppelbindungen ist und
R₂ₛ Wasserstoff ist;
R₃ₛ Wasserstoff oder ein Methylrest ist;
R₄ₛ Wasserstoff; ein Alkylrest mit 1 bis 4 Kohlenstoffatomen; ein Methoxyrest; ein Halogen mit der Atomzahl von 9 bis 35; ein Trifluormethylrest; ein Cyanorest; ein Dimethylaminorest; ein Nitrorest; ein Phenylrest oder ein Benzylrest ist;
R₅ₛ Wasserstoff; ein Alkylrest mit 1 bis 4 Kohlenstoffatomen; ein Methoxyrest; ein Halogen mit der Atomzahl 9 oder 17 oder ein Trifluormethylrest ist und
R₆ₛ Wasserstoff; ein Alkylrest mit 1 bis 3 Kohlenstoffatomen oder ein Methoxyrest ist
mit dem Vorbehalt, daß dann, wenn
i) sowohl R₄ₛ als auch R₅ₛ Trifluormethylreste sind, daß diese dann nicht in ortho-Stellung zueinander sind;
ii) wenn R₁ₛ ein Hydroxyalkylrest ist und jeder der Reste R₄ₛ und R₅ₛ ausgewählt ist aus Wasserstoff und Alkylresten, daß dann mindestens zwei Reste der Reste R₄ₛ, R₅ₛ und R₆ₛ Alkylreste sind und
iii) wenn R₁ₛ ein Alkylrest ist und R₄ₛ ein Phenylrest ist, daß dann R₅ₛ und R₆ₛ etwas anderes als Methoxyreste sind;
je nachdem in freier Form oder Salzform.

5. Pharmazeutische Zusammensetzung umfassend eine Verbindung der Formel I, wie in Anspruch 1 definiert, die die Formel Ia 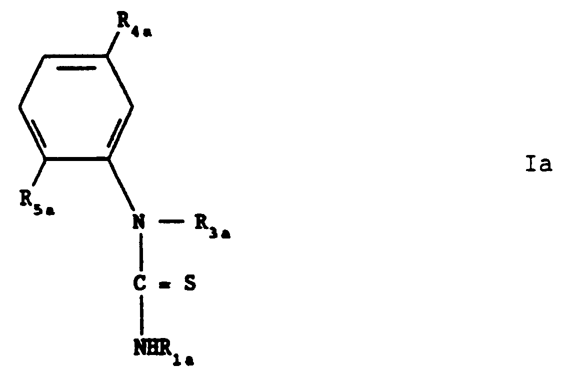 hat, worin
R₁ₐ Wasserstoff; ein Alkylrest mit 3 bis 22 Kohlenstoffatomen; ein Cycloalkylrest mit 3 bis 6 Kohlenstoffatomen, der gegebenenfalls mit Alkylresten mit 1 oder 2 Kohlenstoffatomen mono- oder unabhängig disubstituiert ist; ein Cycloalkylalkylrest mit 3 bis 6 Kohlenstoffatomen im Cycloalkylanteil und 1 bis 3 Kohlenstoffatomen im Alkylenteil; ein Alkoxyalkyl- oder Mercaptoalkylrest mit 2 bis 4 Kohlenstoffatomen im Alkylenteil, 1 bis 3 Kohlenstoffatomen im Alkoxyteil, wobei der Alkoxy- oder Mercaptoteil an einer anderen Position als Position 1 gebunden ist; -CR₇ₐR₈ₐR₉ₐ oder -CH₂CR₇ₐR₈ₐR₉ₐ, worin R₇ₐ und R₈ₐ entweder unabhängig Wasserstoff, Methyl- oder Ethylreste sind oder zusammen einen unverzweigten Alkylenrest mit 3 bis 5 Kohlenstoffatomen bilden und R₉ₐ ein Alkoxycarbonylrest mit insgesamt 2 bis 4 Kohlenstoffatomen oder ein Carbamoylrest ist; ein Alkenylrest mit 4 bis 22 Kohlenstoffatomen, worin die Doppelbindung an einer anderen Position als Position 1 ist, oder ein Alkadienylrest mit 5 bis 22 Kohlenstoffatomen oder ein Alkatrienylrest mit 7 bis 22 Kohlenstoffatomen ist, worin jede Doppelbindung an einer anderen Position als Position 1 ist und kein Kohlenstoffatom Teil von 2 Doppelbindungen ist;
R₃ₐ Wasserstoff oder ein Alkylrest mit 1 bis 3 Kohlenstoffatomen ist;
einer der Reste R₄ₐ und R₅ₐ ein Alkylrest mit 1 bis 4 Kohlenstoffatomen; ein Alkoxyrest mit 1 bis 3 Kohlenstoffatomen; Halogen mit einer Atomzahl von 9 bis 35; ein Trifluormethylrest; Cyanorest; Dialkylaminorest mit unabhängig 1 bis 3 Kohlenstoffatomen in den Alkylanteilen; Nitrorest; Phenylrest oder Benzylrest ist; wobei
der andere der Reste R₄ₐ und R₅ₐ ein Alkylrest mit 1 bis 4 Kohlenstoffatomen; ein Alkoxyrest mit 1 bis 3 Kohlenstoffatomen oder ein Trifluormethylrest ist;
mit dem Vorbehalt, daß dann, wenn
i) sowohl R₄ₐ als auch R₅ₐ Trifluormethylreste sind, daß sie dann nicht in ortho-Stellung zueinander sind und
ii) wenn R₁ₐ ein Alkylrest ist und einer der Reste R₄ₐ und R₅ₐ ein Phenylrest ist, daß dann der andere der Reste R₄ₐ und R₅ₐ etwas anderes als ein Alkoxyrest ist;
je nachdem in freier Form oder Salzform zusammen mit einem pharmazeutisch annehmbaren Träger oder Verdünnungsmittel.

6. Pharmazeutische Zusammensetzung nach Anspruch 5, worin die Verbindung der Formel Ia die Formel Ia' 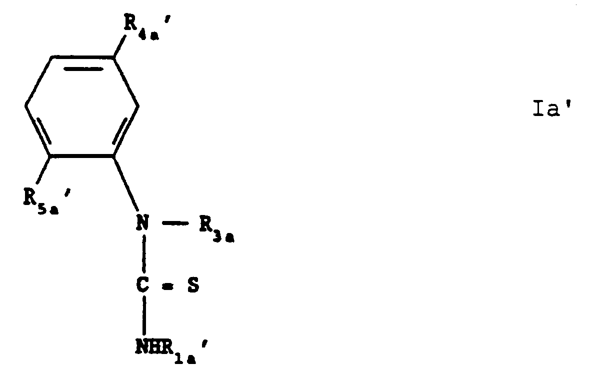 hat, worin
R_{1a'} ein Propyl- oder Isopropylrest oder ein Alkylrest mit 4 bis 22 Kohlenstoffatomen ist, der an einer anderen Position als an dem Kohlenstoffatom, das an das Stickstoffatom gebunden ist, verzweigt ist; ein Cycloalkylrest mit 3 bis 6 Kohlenstoffatomen, der gegebenenfalls mit Alkylresten mit 1 oder 2 Kohlenstoffatomen mono- oder unabhängig disubstituiert ist; ein Cycloalkylalkylrest mit 3 bis 6 Kohlenstoffatomen im Cycloalkylteil und 1 bis 3 Kohlenstoffatomen im Alkylenteil; ein Alkoxyalkyl- oder Mercaptoalkylrest mit 2 bis 4 Kohlenstoffatomen im Alkylenteil, 1 bis 3 Kohlenstoffatomen im Alkoxyteil, wobei der Alkoxy- oder Mercaptoteil an einer anderen Position als Position 1 gebunden ist; -CR₇ₐR₈ₐR₉ₐ oder -CH₂CR₇ₐR₈ₐR₉ₐ, worin R₇ₐ, R₈ₐ und R₉ₐ wie oben definiert sind; ein Alkenylrest mit 4 bis 22 Kohlenstoffatomen, worin die Doppelbindung an einer anderen Position als Position 1 ist, oder ein Alkadienylrest mit 5 bis 22 Kohlenstoffatomen oder ein Alkatrienylrest mit 7 bis 22 Kohlenstoffatomen ist, worin jede Doppelbindung an einer anderen Position als Position 1 ist und kein Kohlenstoffatom Teil von 2 Doppelbindungen ist;
R₃ₐ wie in Anspruch 5 definiert ist;
einer der Reste R_{4a'} und R_{5a'} ein Methylrest; ein Alkoxyrest mit 1 bis 3 Kohlenstoffatomen; ein Halogen mit der Atomzahl von 9 bis 35; ein Trifluormethylrest; Cyanorest; Dialkylaminorest mit unabhängig 1 bis 3 Kohlenstoffatomen in den Alkylteilen; Nitrorest; Phenylrest oder Benzylrest ist;
der andere der Reste R_{4a'} und R_{5a'} ein Methylrest; Alkoxyrest mit 1 bis 3 Kohlenstoffatomen oder Trifluormethylrest ist
mit dem Vorbehalt, daß dann, wenn
i) beide Reste R_{4a'} und R_{5a'} Trifluormethylreste sind, daß sie dann nicht in ortho-Stellung zueinander sind;
ii) wenn R_{1a'} ein Alkylrest ist und einer der Reste R_{4a'} und R_{5a'} ein Phenylrest ist, daß dann der andere der Reste R_{4a'} und R_{5a'} etwas anderes als ein Alkoxyrest ist und
iii) R_{4a'} und R_{5a'} etwas anderes als beide Alkoxyreste sind.

7. Verbindung der Formel I nach Anspruch 1, der die Formel Iaa 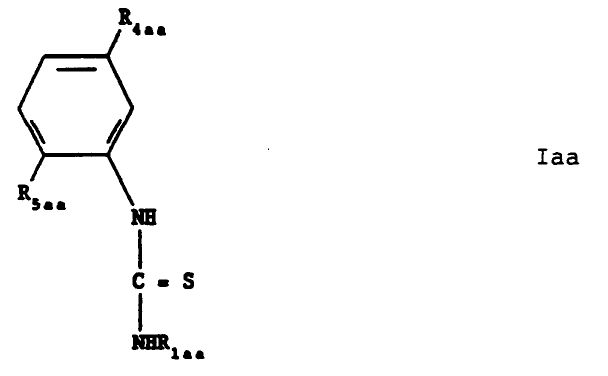 hat, worin
R₁ₐₐ ein Propyl-, Isopropyl- oder 2-Methylpropylrest ist;
R₄ₐₐ Halogen mit einer Atomzahl von 9 bis 35 ist und
R₅ₐₐ ein Alkylrest mit 1 bis 4 Kohlenstoffatomen ist.

8. Verbindung der Formel Iaa nach Anspruch 7, die N-(5-Chlor-2-methylphenyl)-N'-propylthioharnstoff ist.

9. Verbindung der Formel Iaa nach Anspruch 7, die N-(5-Chlor-2-methylphenyl)-N'-2-methylpropylthioharnstoff ist.

10. Verbindung der Formel Iaa nach Anspruch 7, worin R₁ₐₐ, R₄ₐₐ bzw. R₅ₐₐ entweder
- CH(CH₃)₂, 5-Cl bzw. 2-CH₃ oder
- CH₂CH₂CH₃, 5-Cl bzw. 2-CH₂CH₃ oder
- CH₂CH₂CH₃, 5-F bzw. 2-CH₃ oder
- CH₂CH₂CH₃, 5-Br bzw. 2-CH₃ sind.

11. Verbindung der Formel Ia wie in Anspruch 5 definiert, zur Verwendung als Pharmazeutikum.

12. Verbindung nach Anspruch 11 zur Verwendung zur prophylaktischen oder heilenden Behandlung von Atherosklerose oder zur Erhöhung des Blutserumgehaltes an hochdichtem Lipoprotein.

13. Verfahren zur Herstellung einer Verbindung der Formel Ia, wie in Anspruch 7 definiert, umfassend, daß man eine entsprechende Verbindung der Formel IIa 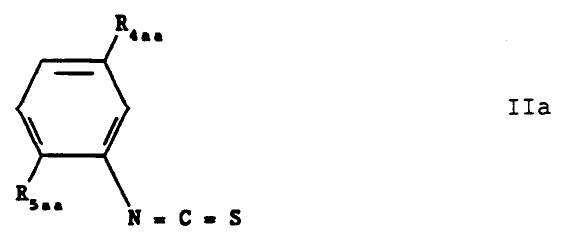 mit einer entsprechenden Verbindung der Formel IIIa
R₁ₐₐNH₂ IIIa
umsetzt und die entstehende Verbindung der Formel I je nachdem in freier Form oder Salzform gewinnt.

## Patentansprüche (Patentansprüche für folgende(n) Vertragsstaat(en): ES, GR)

1. Verfahren zur Herstellung einer Verbindung der Formel I 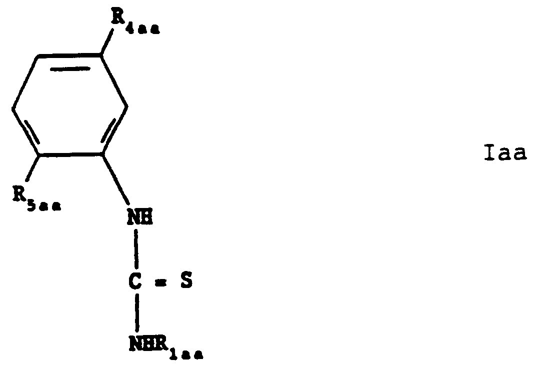 worin
R₁ₐₐ ein Propyl-, Isopropyl- oder 2-Methylpropylrest ist;
R₄ₐₐ Halogen mit einer Atomzahl von 9 bis 35 ist und
R₅ₐₐ ein Alkylrest mit 1 bis 4 Kohlenstoffatomen ist;
je nachdem in freier Form oder Salzform, umfassend, daß man eine entsprechende Verbindung der Formel IIa 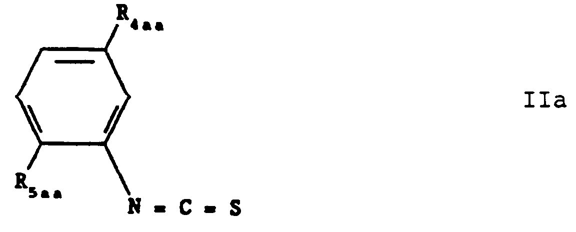 mit einer entsprechenden Verbindung der Formel IIIa
R₁ₐₐNH₂ IIIa
umsetzt und die entstehende Verbindung der Formel I je nachdem in freier Form oder Salzform gewinnt.

2. Verfahren nach Anspruch 1 zur Herstellung einer Verbindung der Formel Iaa, die N-(5-Chlor-2-methylphenyl)-N'-propylthioharnstoff ist.

3. Verfahren nach Anspruch 1 zur Herstellung der Verbindung der Formel Iaa, die N-(5-Chlor-2-methylphenyl)-N'-2-methylpropylthioharnstoff ist.

4. Verfahren nach Anspruch 1 zur Herstellung der Verbindung der Formel Iaa, worin R₁ₐₐ, R₄ₐₐ bzw. R₅ₐₐ entweder
- CH(CH₃)₂, 5-Cl bzw. 2-CH₃ oder
- CH₂CH₂CH₃, 5-Cl bzw. 2-CH₂CH₃ oder
- CH₂CH₂CH₃, 5-F bzw. 2-CH₃ oder
- CH₂CH₂CH₃, 5-Br bzw. 2-CH₃ sind.

5. Verfahren zur Herstellung eines Arzneimittels gegen Atherosklerose oder eines Arzneimittels zur Erhöhung des Blutserumgehaltes an hochdichtem Lipoprotein umfassend, daß man eine Verbindung der Formel Iaa, wie in Anspruch 1 definiert, je nachdem in freier Form oder Salzform, mit mindestens einem pharmazeutisch annehmbaren Träger oder Verdünnungsmittel vermischt.

## Revendications (Revendications pour l'(les) Etat(s) contractant(s) suivant(s): AT, BE, CH, LI, DE, DK, FR, GB, IT, LU, NL, PT, SE)

1. L'utilisation d'un composé de formule I 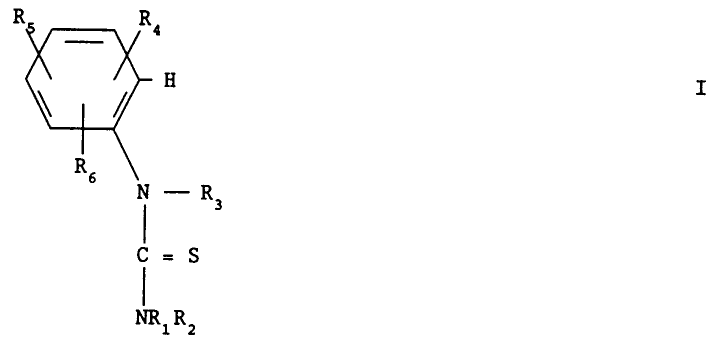 dans laquelle
R₁ signifie l'hydrogène; ou un groupe alkyle contenant de 1 à 22 atomes de carbone; cycloalkyle contenant de 3 à 6 atomes de carbone éventuellement mono- ou indépendamment disubstitué par un groupe alkyle contenant 1 ou 2 atomes de carbone; cycloalkylalkyle contenant de 3 à 6 atomes de carbone dans la partie cycloalkyle et de 1 à 3 atomes de carbone dans la partie alkylène ou phénylalkyle contenant au total de 7 à 9 atomes de carbone; hydroxyalkyle, alcoxyalkyle ou mercaptoalkyle contenant de 2 à 4 atomes de carbone dans la partie alkylène, de 1 à 3 atomes de carbone dans la partie alcoxy, et avec la partie hydroxy, alcoxy ou mercapto fixée en une position autre que la position 1; -CR₇R₈R₉ ou -CH₂R₇R₈R₉ où R₇ et R₈ soit signifient indépendamment l'hydrogène ou un groupe méthyle ou éthyle ou bien signifient ensemble un groupe alkylène non ramifié contenant de 3 à 5 atomes de carbone et R₉ signifie un groupe carboxy, alcoxycarbonyle contenant au total de 2 à 4 atomes de carbone ou carbamoyle; alcényle contenant de 3 à 22 atomes de carbone où la double liaison est située en une position autre que la position 1 ou alcadiényle contenant de 5 à 22 atomes de carbone ou alcatriényle contenant de 7 à 22 atomes de carbone où chaque double liaison est située en une position autre que la position 1 et aucun atome de carbone ne fait partie des deux doubles liaisons, et
R₂ signifie l'hydrogène;
R₃ signifie l'hydrogène ou un groupe alkyle contenant de 1 à 3 atomes de carbone;
R₄ signifie l'hydrogène; ou un groupe alkyle contenant de 1 à 4 atomes de carbone; alcoxy contenant de 1 à 3 atomes de carbone; un halogène ayant un nombre atomique de 9 à 35; un groupe trifluorométhyle; cyano; dialkylamino contenant indépendamment de 1 à 3 atomes de carbone dans les parties alkyle; nitro; phényle; ou benzyle;
R₅ signifie l'hydrogène; ou un groupe alkyle contenant de 1 à 4 atomes de carbone; alcoxy contenant de 1 à 3 atomes de carbone; un halogène ayant un nombre atomique de 9 ou 17; ou un groupe trifluorométhyle; et
R₆ signifie l'hydrogène; ou un groupe alkyle contenant de 1 à 3 atomes de carbone; ou alcoxy contenant de 1 à 3 atomes de carbone;
avec les conditions que:
i) lorsque les deux symboles R₄ et R₅ signifient un groupe trifluorométhyle, alors ils ne sont pas situés en position ortho l'un par rapport à l'autre;
ii) lorsque R₁ signifie un groupe hydroxyalkyle et chaque symbole R₄, R₅ et R₆ est choisi parmi l'hydrogène ou un groupe alkyle,
alors au moins deux des symboles R₄, R₅ et R₆ signifient un groupe alkyle;
et
iii) lorsque R₁ signifie un groupe alkyle et R₄ signifie un groupe phényle,
alors R₅ et R₆ ont une signification autre qu'un groupe alcoxy;
sous forme libre ou, lorsque cela est approprié, sous forme d'un sel, pour la préparation d'un médicament contre l'athérosclérose ou d'un médicament pour l'augmentation du taux de lipoprotéine à densité élevée dans le sérum sanguin.

2. Un procédé de préparation d'un médicament contre l'athérosclérose ou d'un médicament pour l'augmentation du taux de lipoprotéine à densité élevée dans le sérum sanguin qui comprend le mélange d'un composé de formule I tel que défini à la revendication 1 sous forme libre ou, lorsque cela est approprié, sous forme d'un sel, avec au moins un véhicule ou diluant pharmaceutiquement acceptable.

3. L'utilisation selon la revendication 1 ou le procédé selon la revendication 2 où le composé de formule I est tel que défini à la revendication 1 avec la condition supplémentaire que R₄ ait une signification autre qu'un groupe benzyle et R₅ ait une signification autre qu'un groupe alkyle contenant 4 atomes de carbone.

4. L'utilisation selon la revendication 1 ou le procédé selon la revendication 2 où le composé de formule I signifie un composé de formule Is 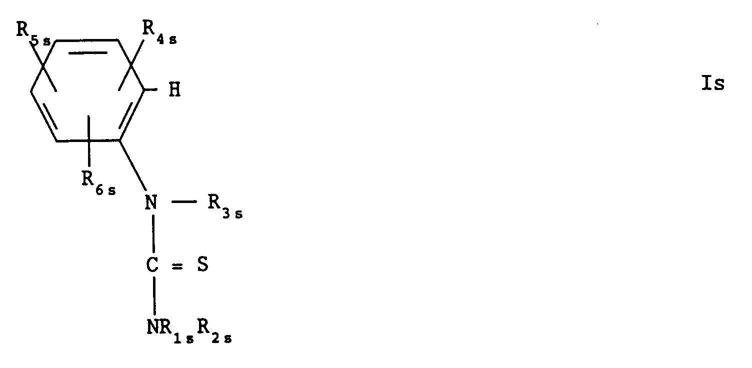 dans laquelle
R₁ₛ signifie l'hydrogène; ou un groupe alkyle contenant de 1 à 6 atomes de carbone; cycloalkyle contenant de 3 à 6 atomes de carbone éventuellement monosubstitué par un groupe méthyle; cyclopropylméthyle; phénylalkyle contenant 7 ou 8 atomes de carbone; hydroxyalkyle contenant de 2 à 4 atomes de carbone, méthoxyéthyle ou mercaptoéthyle, avec la partie hydroxy, méthoxy ou mercapto fixée en une position autre que la position 1; -CR₇ₛR₈ₛR₉ₛ ou -CH₂R₇ₛR₈ₛR₉ₛ où R₇ₛ et R₈ₛ soit signifient indépendamment l'hydrogène ou un groupe méthyle ou bien ils signifient ensemble un groupe alkylène non ramifié contenant de 3 à 5 atomes de carbone et R₉ₛ signifie un groupe carboxy, méthoxycarbonyle ou carbamoyle; alcényle contenant de 3 à 18 atomes de carbone où la double liaison est situé en une position autre que la position 1, ou bien un groupe alcadiényle contenant de 5 à 18 atomes de carbone ou alcatriényle contenant de 7 à 18 atomes de carbone où chaque double liaison est située en une position autre que la position 1 et aucun atome de carbone ne fait partie des deux doubles liaisons, et
R₂ₛ signifie l'hydrogène;
R₃ₛ signifie l'hydrogène ou un groupe méthyle;
R₄ₛ signifie l'hydrogène; ou un groupe alkyle contenant de 1 à 4 atomes de carbone; méthoxy; un halogène ayant un nombre atomique de 9 à 35; un groupe trifluorométhyle; cyano; diméthylamino; nitro; phényle; ou benzyle;
R₅ₛ signifie l'hydrogène; un groupe alkyle contenant de 1 à 4 atomes de carbone; méthoxy; un halogène ayant un nombre atomique de 9 ou 17; ou un groupe trifluorométhyle; et
R₆ₛ signifie l'hydrogène; ou un groupe alkyle contenant de 1 à 3 atomes de carbone; ou un groupe méthoxy;
avec les conditions que
i) lorsque les deux symboles R₄ₛ et R₅ₛ signifient un groupe trifluorométhyle,
alors ils ne sont pas situés en position ortho l'un par rapport à l'autre;
ii) lorsque R₁ₛ signifie un groupe hydroxyalkyle et chaque symbole R₄ₛ et R₅ₛ est choisi parmi l'hydrogène et un groupe alkyle,
alors au moins deux des symboles R₄ₛ, R₅ₛ et R₆ₛ signifient un groupe alkyle;
et
iii) lorsque R₁ₛ signifie un groupe alkyle et R₄ₛ signifie un groupe phényle,
alors R₅ₛ et R₆ₛ ont une signification autre qu'un groupe méthoxy;
sous forme libre ou, lorsque cela est approprié, sous forme d'un sel.

5. Une composition pharmaceutique comprenant un composé de formule I tel que défini à la revendication 1 qui répond à la formule Ia 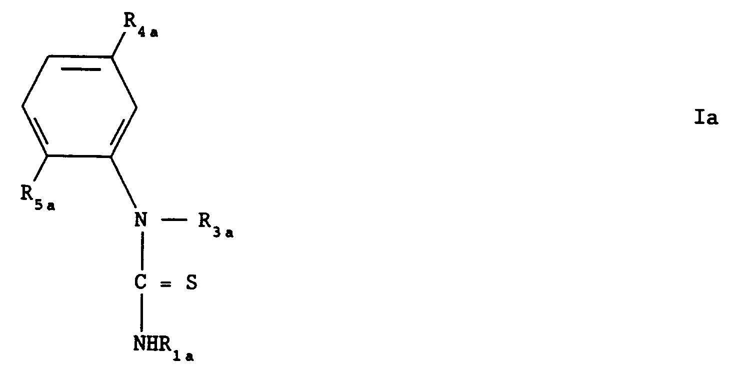 dans laquelle
R₁ₐ signifie l'hydrogène; ou un groupe alkyle contenant de 3 à 22 atomes de carbone; cycloalkyle contenant de 3 à 6 atomes de carbone éventuellement mono- ou indépendamment disubstitué par un groupe alkyle contenant 1 ou 2 atomes de carbone; cycloalkylalkyle contenant de 3 à 6 atomes de carbone dans la partie cycloalkyle et de 1 à 3 atomes de carbone dans la partie alkylène; alcoxyalkyle ou mercaptoalkyle contenant de 2 à 4 atomes de carbone dans la partie alkylène, de 1 à 3 atomes de carbone dans la partie alcoxy et avec la partie alcoxy ou mercapto fixée en une position autre que la position 1; -CR₇ₐR₈ₐR₉ₐ ou -CH₂R₇ₐR₈ₐR₉ₐ où R₇ₐ et R₈ₐ soit signifient indépendamment l'hydrogène ou un groupe méthyle ou éthyle ou bien signifient ensemble un groupe alkylène non ramifié contenant de 3 à 5 atomes de carbone et R₉ₐ signifie un groupe alcoxycarbonyle contenant au total de 2 à 4 atomes de carbone ou un groupe carbamoyle; alcényle contenant de 4 à 22 atomes de carbone où la double liaison est située en une position autre que la position 1, ou alcadiényle contenant de 5 à 22 atomes de carbone ou alcatriényle contenant de 7 à 22 atomes de carbone où chaque double liaison est située en une position autre que la position 1 et aucun atome de carbone ne fait partie des deux doubles liaisons, et
R₃ₐ signifie l'hydrogène ou un groupe alkyle contenant de 1 à 3 atomes de carbone;
l'un des symboles R₄ₐ et R₅ₐ signifie un groupe alkyle contenant de 1 à 4 atomes de carbone; alcoxy contenant de 1 à 3 atomes de carbone; un halogène ayant un nombre atomique de 9 à 35; un groupe trifluorométhyle; cyano; dialkylamino contenant indépendamment de 1 à 3 atomes de carbone dans les parties alkyle; nitro; phényle ou benzyle;
l'autre symbole parmi R₄ₐ et R₅ₐ signifie un groupe alkyle contenant de 1 à 4 atomes de carbone; alcoxy contenant de 1 à 3 atomes de carbone; ou bien trifluorométhyle;
avec les conditions que
i) lorsque les deux symboles R₄ₐ et R₅ₐ signifient un groupe trifluorométhyle, alors ils ne sont pas situés en position ortho l'un par rapport à l'autre; et
ii) lorsque R₁ₐ signifie un groupe alkyle et l'un des symboles R₄ₐ et R₅ₐ signifie un groupe phényle,
alors l'autre symbole parmi R₄ₐ et R₅ₐ a une signification autre qu'un groupe alcoxy;
sous forme libre ou, lorsque cela est approprié, sous forme d'un sel, ensemble avec un véhicule ou diluant pharmaceutiquement acceptable.

6. Une composition pharmaceutique selon la revendication 5, caractérisée en ce que le composé de formule Ia répond à la formule Ia' 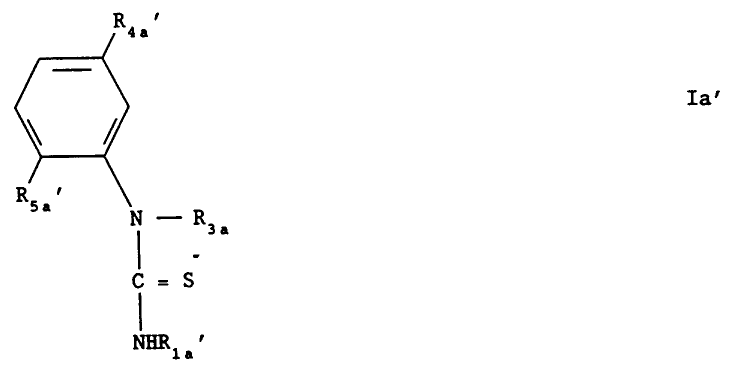 dans laquelle
R₁ₐ' signifie un groupe propyle ou isopropyle ou alkyle contenant de 4 à 22 atomes de carbone ramifié en une position autre que celle de l'atome de carbone fixé à l'atome d'azote; cycloalkyle contenant de 3 à 6 atomes de carbone éventuellement mono- ou indépendamment disubstitué par un groupe alkyle contenant 1 ou 2 atomes de carbone; cycloalkylalkyle contenant de 3 à 6 atomes de carbone dans la partie cycloalkyle et de 1 à 3 atomes de carbone dans la partie alkylène; alcoxyalkyle ou mercaptoalkyle contenant de 2 à 4 atomes de carbone dans la partie alkylène, de 1 à 3 atomes de carbone dans la partie alcoxy et avec la partie alcoxy ou mercapto fixée en une position autre que la position 1; -CR₇ₐR₈ₐR₉ₐ ou -CH₂R₇ₐR₈ₐR₉ₐ où R₇ₐ, R₈ₐ et R₉ₐ sont tels que définis plus haut; alcényle contenant de 4 à 22 atomes de carbone où la double liaison est située en une position autre que la position 1, ou alcadiényle contenant de 5 à 22 atomes de carbone ou alcatriényle contenant de 7 à 22 atomes de carbone où chaque double liaison est située en une position autre que la position 1 et aucun atome de carbone ne fait partie des deux doubles liaisons,
R₃ₐ est tel que défini à la revendication 5;
l'un des symboles R₄ₐ' et R₅ₐ' signifie un groupe méthyle; alcoxy contenant de 1 à 3 atomes de carbone; un halogène ayant un nombre atomique de 9 à 35; un groupe trifluorométhyle; cyano; dialkylamino contenant indépendamment de 1 à 3 atomes de carbone dans les parties alkyle; nitro; phényle; ou benzyle;
l'autre symbole parmi R₄ₐ' et R₅ₐ' signifie un groupe méthyle; alcoxy contenant de 1 à 3 atomes de carbone; ou trifluorométhyle;
avec les conditions que
i) lorsque les deux symboles R₄ₐ' et R₅ₐ' signifient un groupe trifluorométhyle, alors ils ne sont pas situés en position ortho l'un par rapport à l'autre;
ii) lorsque R₁ₐ' signifie un groupe alkyle et l'un des symboles R₄ₐ' et R₅ₐ' signifie un groupe phényle,
alors l'autre symbole parmi R₄ₐ' et R₅ₐ' a une signification autre qu'un groupe alcoxy, et
iii) R₄ₐ' et R₅ₐ' ont tous les deux une signification autre qu'un groupe alcoxy.

7. Un composé de formule I tel que défini à la revendication 1 qui répond à la formule Iaa 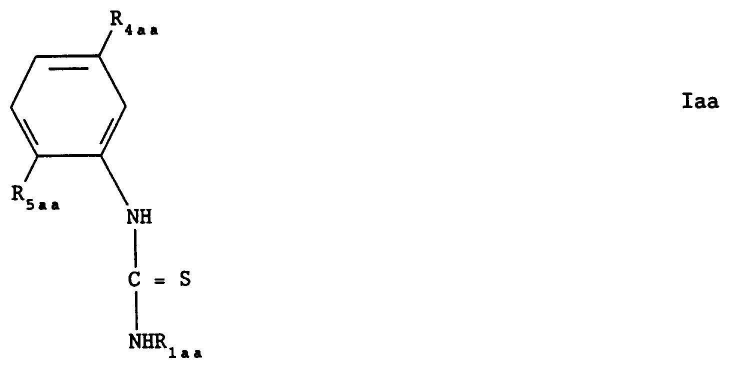 dans laquelle
R₁ₐₐ signifie un groupe propyle, isopropyle ou 2-méthylpropyle;
R₄ₐₐ signifie un halogène ayant un nombre atomique de 9 à 35, et
R₅ₐₐ signifie un groupe alkyle contenant de 1 à 4 atomes de carbone.

8. Le composé de formule Iaa selon la revendication 7 qui est la N-(5-chloro-2-méthylphényl)-N'-propylthiourée.

9. Le composé de formule Iaa selon la revendication 7 qui est la N-(5-chloro-2-méthylphényl)-N'-2-méthylpropylthiourée.

10. Le composé de formule Iaa selon la revendication 7 où R₁ₐₐ, R₄ₐₐ et R₅ₐₐ signifient respectivement, soit
- CH(CH₃)₂, 5-Cl et 2-CH₃, ou bien
- CH₂CH₂CH₃, 5-Cl et 2-CH₂CH₃, ou bien
- CH₂CH₂CH₃, 5-F et 2-CH₃, ou bien
- CH₂CH₂CH₃, 5-Br et 2-CH₃.

11. Un composé de formule Ia tel que défini à la revendication 5 pour une utilisation comme médicament.

12. Un composé selon la revendication 11 pour une utilisation dans le traitement prophylactique ou curatif de l'athérosclérose ou pour augmenter le taux de lipoprotéine à densité élevée dans le sérum sanguin.

13. Un procédé de préparation d'un composé de formule Iaa tel que défini à la revendication 7 qui comprend la réaction d'un composé correspondant de formule IIa 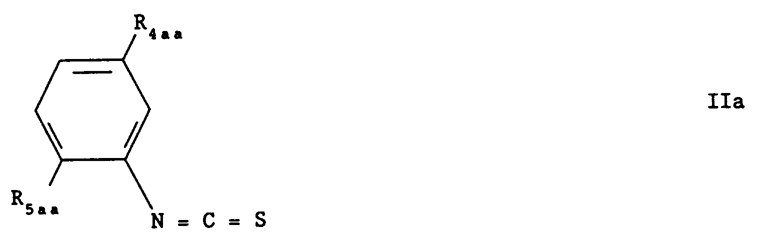 avec un composé correspondant de formule IIIa
R₁ₐₐNH₂ (IIIa)
et la récupération du composé résultant de formule I sous forme libre ou, lorsque cela est approprié, sous forme d'un sel.

## Revendications (Revendications pour l'(les) Etat(s) contractant(s) suivant(s): ES, GR)

1. Un procédé de préparation d'un composé de formule I 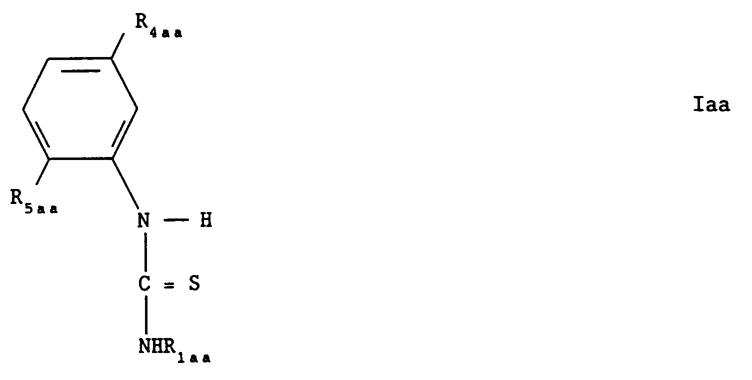 dans laquelle
R₁ₐₐ signifie un groupe propyle, isopropyle ou 2-méthylpropyle;
R₄ₐₐ signifie un halogène ayant un nombre atomique de 9 à 35, et
R₅ₐₐ signifie un groupe alkyle contenant de 1 à 4 atomes de carbone,
sous forme libre ou, lorsque cela est approprié, sous forme d'un sel, qui comprend la réaction d'un composé correspondant de formule IIa 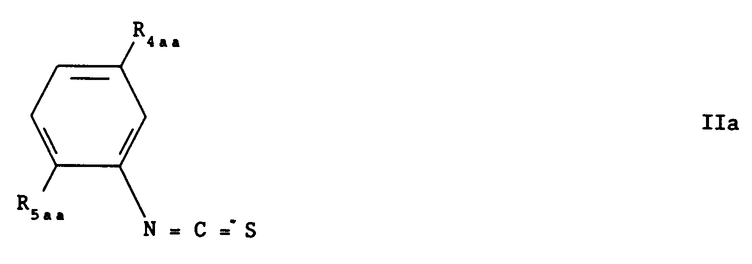 avec un composé correspondant de formule IIIa
R₁ₐₐNH₂ (IIIa)
et la récupération du composé résultant de formule I sous forme libre ou, lorsque cela est approprié, sous forme d'un sel.

2. Un procédé selon la revendication 1 pour la préparation du composé de formule Iaa qui est la N-(5-chloro-2-méthylphényl)-N'-propylthiourée.

3. Un procédé selon la revendication 1 pour la préparation du composé de formule Iaa qui est la N-(5-chloro-2-méthylphényl)-N'-2-méthylpropylthiourée.

4. Un procédé selon la revendication 1 pour la préparation du composé de formule Iaa où R₁ₐₐ, R₄ₐₐ et R₅ₐₐ signifient respectivement soit
- CH(CH₃)₂, 5-Cl et 2-CH₃, ou bien
- CH₂CH₂CH₃, 5-Cl et 2-CH₂CH₃, ou bien
- CH₂CH₂CH₃, 5-F et 2-CH₃, ou bien
- CH₂CH₂CH₃, 5-Br et 2-CH₃.

5. Un procédé de préparation d'un médicament contre l'athérosclérose ou d'un médicament pour augmenter le taux de lipoprotéine à densité élevée dans le sérum sanguin, qui comprend le mélange d'un composé de formule Iaa tel que défini à la revendication 1 sous forme libre ou, lorsque cela est approprié, sous forme d'un sel, avec au moins un véhicule ou diluant pharmaceutiquement acceptable.
